(19) Europäisches Patentamt
European Patent Office
Office européen des brevets

(11) Publication number: **0 417 840 B1**

# (12) EUROPEAN PATENT SPECIFICATION

(45) Date of publication of patent specification: **07.12.94**

(51) Int. Cl.$^5$: **C07D 487/06**, A61K 31/55, C07D 243/14, //(C07D487/06, 243:00,235:00)

(21) Application number: **90202369.6**

(22) Date of filing: **06.09.90**

Consolidated with 90913194.8/0491751 (European application No./publication No.) by decision dated 15.07.92.

## (54) Antiviral tetrahydroimidazo [1,4]-benzodiazepines.

(30) Priority: **13.09.89 US 406625**

(43) Date of publication of application:
**20.03.91 Bulletin 91/12**

(45) Publication of the grant of the patent:
**07.12.94 Bulletin 94/49**

(84) Designated Contracting States:
**AT BE CH DE DK ES FR GB GR IT LI LU NL SE**

(56) References cited:
**EP-A- 0 336 466**
**EP-A- 0 384 522**

**NATURE, vol. 343, 1st February 1990, pages 470-474, London, GB; R. PAUWELS et al.: "Potent and selective inhibition of HIV-1 replication in vitro by a novel series of TIBO dervatives"**

(73) Proprietor: **JANSSEN PHARMACEUTICA N.V.**
**Turnhoutseweg 30**
**B-2340 Beerse (BE)**

(72) Inventor: **Kukla, Michael Joseph**
**1551 Oak Hollow Drive**
**Maple Glen**
**Pennsylvania (US)**
Inventor: **Breslin, Henry Joseph**
**11974 Muhlenburg Drive**
**Lansdale**
**Pennsylvania (US)**
Inventor: **Raeymaekers, Alfons Herman Margaretha**
**Aanbeeldstraat 1**
**B-2340 Beerse (BE)**
Inventor: **Van Gelder, Josephus Ludovicus Hubertus**
**Bareelstraat 30**
**B-2450 Kasterlee (BE)**
Inventor: **Janssen, Paul Adriaan Jan**
**Antwerpsesteenweg 20**
**B-2350 Vosselaar (BE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3.10/3.09/3.3.3)

## Description

Background of the invention

In the Eur. J. Med. Chem. 1978, 13, 53-59, there are described three tetrahydroimidazo[4,5,1-jk][1,4]-benzodiazepines. EP-A-0,336,466, published October 11, 1989 discloses antiviral tetrahydroimidazo[1,4]-benzodiazepinones. In Nature, 343, 470 (1990), there are described the same tetrahydroimidazo[1,4]-benzodiazepinones and a few corresponding thiones. The present compounds differ therefrom by the fact that the 7-position is invariably substituted with at least one alkyl group and by their favourable pharmacological properties.

Description of the invention

The present invention is concerned with tetrahydroimidazo[1,4]benzodiazepines having the formula :

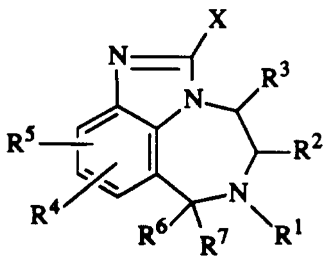

(I),

the pharmaceutically acceptable acid addition salts and the stereochemically isomeric forms thereof, wherein

$R^1$ is $C_{1-6}$alkyl optionally substituted with aryl; $C_{3-6}$alkynyl; $C_{3-6}$cycloalkyl; or a radical of formula :

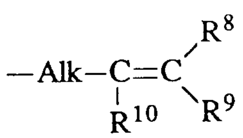

(a-1);

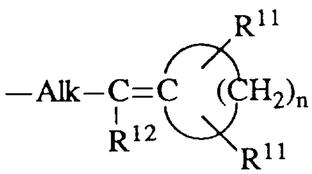

(a-2);

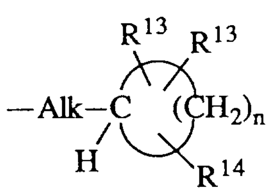

(a-3) or

$—Alk—S(O)_m—R^{15}$ (a-4);

Alk is $C_{1-6}$alkanediyl;
$R^8$ and $R^9$ each independently are hydrogen, halo, $C_{3-6}$cycloalkyl, trifluoromethyl, 2,2,2-trifluoroethyl, $C_{1-4}$alkyl optionally substituted with $C_{1-4}$alkyloxy;
$R^{10}$ is hydrogen, halo or $C_{1-4}$alkyl;
each $R^{11}$ independently is hydrogen or $C_{1-4}$alkyl; or both $R^{11}$ taken together may form a $C_{1-6}$alkanediyl radical;
$R^{12}$ is hydrogen, halo or $C_{1-4}$alkyl;
n is 2, 3, 4, 5 or 6;

each $R^{13}$ independently is hydrogen or $C_{1-4}$alkyl; or both $R^{13}$ taken together may form a $C_{1-6}$alkanediyl radical;
$R^{14}$ is hydrogen or $C_{2-6}$alkenyl;
m is 0, 1 or 2;
$R^{15}$ is $C_{1-6}$alkyl, aryl, arylmethyl, $C_{3-6}$cycloalkyl or ($C_{3-6}$cycloalkyl)$C_{1-4}$alkyl;

| | |
|---|---|
| $R^2$ | is hydrogen or $C_{1-6}$alkyl; |
| $R^3$ | is hydrogen or $C_{1-6}$alkyl; |
| $R^4$ and $R^5$ | each independently are hydrogen, $C_{1-6}$alkyl, halo, cyano, nitro, trifluoromethyl, hydroxy, $C_{1-6}$alkyloxy, amino, mono-or di($C_{1-6}$alkyl)amino, $C_{1-6}$alkylcarbonylamino or arylcarbonylamino; |
| $R^6$ | is $C_{1-6}$alkyl; |
| $R^7$ | is hydrogen; |
| X | is OH, SH or $NR^{16}R^{17}$; |
| $R^{16}$ | is hydrogen, $C_{1-6}$alkyl, aryl, cyano, hydroxy, amino, nitro, $C_{1-6}$alkyloxycarbonyl, $C_{1-6}$alkylcarbonyl, $C_{1-6}$alkylsulfonyl or arylsulfonyl; |
| $R^{17}$ | is hydrogen, $C_{1-6}$alkyl or aryl; and |

each aryl is phenyl optionally substituted with from 1 to 3 substituents independently selected from $C_{1-6}$alkyl, halo, hydroxy, $C_{1-6}$alkyloxy, amino, nitro and trifluoromethyl.

The compounds of formula (I) may also exist in their tautomeric form. Said tautomeric form, although not explicitly indicated in the above formula, is intended to be included within the scope of the present invention.

In the foregoing definitions the term halo is generic to fluoro, chloro, bromo and iodo; $C_{1-4}$alkyl defines straight and branch chained saturated hydrocarbon radicals having from 1 to 4 carbon atoms such as, for example, methyl, ethyl, propyl, 1-methylethyl, butyl, 1-methylpropyl, 2-methylpropyl, 1,1-dimethylethyl and the like; $C_{1-6}$alkyl defines $C_{1-4}$alkyl radicals as defined hereinabove and the higher homologs thereof having from 5 to 6 carbon atoms; $C_{1-6}$alkanediyl defines bivalent straight or branch chained hydrocarbon radicals containing 1 to 6 carbon atoms such as, for example, 1,2-ethanediyl, 1,3-propanediyl, 1,4-butanediyl, 1,5-pentanediyl, 1,6-hexanediyl and the branched isomers thereof; $C_{2-6}$alkenyl defines straight and branched hydrocarbon radicals containing one double bond and having from 2 to 6 carbon atoms such as, for example, ethenyl, 2-propenyl, 2-butenyl, 3-butenyl, 2-methyl-2-propenyl, pentenyl, hexenyl and the like; $C_{3-6}$alkynyl defines straight and branch chained hydrocarbon radicals containing a triple bond and having from 3 to 6 carbon atoms such as, for example, 2-propynyl, 2-butynyl, 3-butynyl, pentynyl, hexynyl and the like ; $C_{3-6}$cycloalkyl defines cyclopropyl, cyclobutyl, cyclopentyl and cyclohexyl.

Each $R^{11}$, $R^{13}$ and $R^{14}$ in the radicals of formula (a-2) and (a-3), when being as defined hereinbefore but other than hydrogen, is meant to replace a hydrogen atom of the $-(CH_2)_n-$ or the -CH- moiety in said radicals.

Depending on the nature of the various substituents, the compounds of formula (I) may have several asymmetric carbon atoms . Unless otherwise mentioned or indicated, the chemical designation of compounds denotes the mixture of all possible stereochemically isomeric forms, said mixtures containing all diastereomers and enantiomers of the basic molecular structure. The absolute configuration of each chiral center may be indicated by the stereochemical descriptors R and S, this R and S notation corresponding to the rules described in Pure Appl. Chem. 1976, 45, 11-30. Stereochemically isomeric forms of the compounds of formula (I) are obviously intended to be embraced within the scope of the invention.

Pure stereochemically isomeric forms of the compounds of formula (I) may be obtained by the application of art-known procedures. Diastereoisomers may be separated by physical separation methods such as selective crystallization and chromatographic techniques, e.g., counter current distribution, liquid chromatography and the like; and enantiomers may be separated from each other by the selective crystallization of their diastereomeric salts with optically active acids. Pure stereochemically isomeric forms may also be derived from the corresponding pure stereochemically isomeric forms of the appropriate starting materials, provided that the reaction occurs stereospecifically.

The compounds of formula (I) have basic properties and, consequently, they may be converted to their therapeutically active non-toxic acid addition salt forms by treatment with appropriate acids, such as, for example, inorganic acids, e.g. hydrochloric, hydrobromic and the like acids, sulfuric acid, nitric acid, phosphoric acid and the like; or organic acids, such as, for example, acetic, propanoic, hydroxyacetic, 2-hydroxypropanoic, 2-oxopropanoic, ethanedioic, propanedioic, butanedioic, (Z)-2-butenedioic, (E)-2-butenedioic, 2-hydroxybutanedioic, 2,3-dihydroxybutanedioic, 2-hydroxy-1,2,3-propanetricarboxylic, methanesulfonic, ethanesulfonic, benzenesulfonic, 4-methylbenzenesulfonic, cyclohexanesulfamic, 2-hydroxybenzoic, 4-amino-2-hydroxybenzoic and the like acids. Conversely the salt form be converted by treatment

with alkali into the free base form. The term pharmaceutically acceptable acid addition salts also comprises the solvates which the compounds of formula (I) may form and said solvates are intended to be included within the scope of the present invention. Examples of such solvates are e.g. the hydrates, alcoholates and the like.

An interesting group of compounds of formula (I) are those wherein $R^1$ is $C_{1-6}$alkyl, $C_{3-6}$alkenyl, $C_{3-6}$alkynyl, $C_{3-6}$cycloalkyl or $C_{1-6}$alkyl substituted with aryl or $C_{3-6}$cycloalkyl; $R^4$ and $R^5$ each independently are hydrogen, $C_{1-6}$alkyl, halo, cyano, nitro, trifluoromethyl, hydroxy, $C_{1-6}$alkyloxy, amino or mono- or di($C_{1-6}$alkyl)amino; $R^7$ is hydrogen; and $R^2$, $R^3$, X, $R^{16}$, $R^{17}$ and aryl are as defined under formula (I).

Particular compounds are those compounds of formula (I) or those compounds comprised within the abovementioned interesting group, wherein $R^1$ is $C_{1-6}$alkyl optionally substituted with aryl, $C_{3-6}$alkynyl or a radical of formula (a-1), (a-2) or (a-3); and/or $R^4$ and $R^5$ each independently are hydrogen, $C_{1-6}$alkyl, halo, cyano, nitro, trifluoromethyl, hydroxy or $C_{1-6}$alkyloxy.

More particular compounds are those particular compounds wherein $R^1$ is $C_{3-6}$alkyl or a radical of formula (a-1) or (a-3); and/or $R^5$ is hydrogen; and/or $R^6$ is $C_{1-4}$alkyl; and/or $R^7$ is hydrogen.

A first particular subgroup comprises those more particular compounds wherein $R^2$ and $R^3$ each independently are hydrogen or methyl; and/or X is OH or SH.

A second particular subgroup comprises those more particular compounds wherein $R^2$ and $R^3$ each independently are hydrogen or methyl; and/or X is $NR^{16}R^{17}$ and $R^{17}$ is hydrogen.

Interesting compounds within the first particular subgroup are those wherein $R^1$ is $C_{3-6}$alkyl or a radical of formula (a-1) wherein $R^8$ and $R^9$ each independently are $C_{3-6}$cycloalkyl, trifluoromethyl or $C_{1-4}$alkyl; or a radical of formula (a-2) wherein $R^{12}$ is hydrogen or $C_{1-4}$alkyl; or a radical of formula (a-3) wherein n is 2 or 3.

Particularly interesting compounds are those interesting compounds wherein $R^1$ is propyl; methylcyclopropyl optionally substituted with one or two methyl groups and/or one 2-methylpropenyl group; methylcyclobutyl; 2-propenyl; 2-butenyl; 2-methyl-2-butenyl; 3-methyl-2-butenyl, 2,3-dimethyl-2-butenyl or 3-ethyl-2-pentenyl; and/or $R^4$ is hydrogen, methyl or chloro; and/or $R^6$ is methyl.

The most interesting compound is trans-4,5,6,7-tetrahydro-5,7-dimethyl-6-(3-methyl-2-butenyl)imidazo-[4,5,1-jk][1,4]benzodiazepine-2(1H)-thione.

The compounds of formula (I) can generally be prepared by reacting a 4,5,6,7-tetrahydroimidazo[4,5,1-jk][1,4] benzodiazepine derivative of formula (II) with a reagent of formula B-X (III), wherein X is as defined hereinabove.

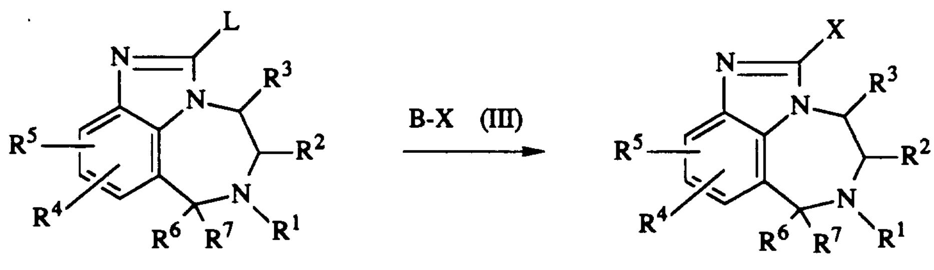


(II) (I)

In formula (II), L is a reactive leaving group such as, for example, halo, e.g. chloro, bromo. Appropriate reagents of formula B-X (III) are, for example, water, thiourea, an alkali metal thiosulfate, e.g. sodium thiosulfate, ammonia, mono- and di($C_{1-6}$alkyl)amines, mono- and di(aryl)amines, ($C_{1-6}$alkyl)(aryl)amines, hydroxylamine, hydrazine and the like reagents. Said reaction can conveniently be conducted by stirring and optionally heating the reactants in a reaction-inert solvent such as, for example, water, an alkanol, e.g., methanol, ethanol, 1-propanol, 2-propanol, butanol, 1,2-ethanediol and the like; or an aromatic hydrocarbon, e.g. benzene, methylbenzene, dimethylbenzene and the like; a halogenated hydrocarbon, e.g. trichloromethane, tetrachloromethane, chlorobenzene and the like; an ether, e.g. tetrahydrofuran, 1,4-dioxane, 1,1'-oxybisbutane, 1,1'-oxybis(2-methoxyethane), 1,2-bis(2-methoxyethoxy)ethane and the like; a dipolar aprotic solvent, e.g. N,N-dimethylformamide, N,N-dimethylacetamide, dimethyl sulfoxide, 1-methyl-2-pyrrolidinone, pyridine, methylpyridine, dimethylpyridine, tetrahydrothiophene 1,1-dioxide and the like; or a mixture of such solvents. In some cases it may be appropriate to conduct said reaction in an excess of the reagent of formula (III), optionally in the presence of a reaction-inert solvent as defined above. In particular, the reaction may be conducted at an elevated temperature, more particularly the reflux temperature of the

reaction mixture. Further, it may be appropriate to add to the reaction mixture a base such as, for example, an amine, e.g. N,N-diethylethanamine, N-ethyl-N-(1-methylethyl)-2-propanamine, 4-methylmorpholine and the like amines.

The compounds of formula (I) may also be obtained by N-alkylating an intermediate of formula (V) with a reagent of formula $R^1$-W (IV) wherein W represents an appropriate reactive leaving group such as, for example, halo, e.g. chloro, bromo or iodo; or a sulfonyloxy group, e.g. benzenesulfonyloxy, 4-methylbenzenesulfonyloxy, methanesulfonyloxy and the like.

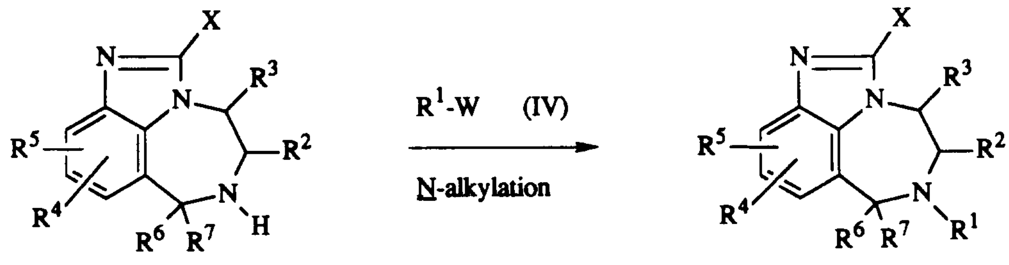


(V) (I)

Said N-alkylation reaction may conveniently be conducted in a reaction-inert solvent such as, for example, an aromatic hydrocarbon, e.g., benzene, methylbenzene, dimethylbenzene and the like; a lower alkanol, e.g., methanol, ethanol, 1-butanol and the like; a ketone, e.g., 2-propanone, 4-methyl-2-pentanone and the like; an ether, e.g., 1,4-dioxane, 1,1'-oxybisethane, tetrahydrofuran and the like; a dipolar aprotic solvent, e.g. N,N-dimethylformamide, N,N-dimethylacetamide, nitrobenzene, dimethyl sulfoxide, 1-methyl-2-pyrrolidinone, and the like, or a mixture of such solvents. The addition of an appropriate base such as, for example, an alkali metal carbonate or hydrogen carbonate, e.g. sodium carbonate, sodium hydrogen carbonate; sodium hydride or an organic base such as, for example, N,N-diethylethanamine or N-(1-methylethyl)-2-propanamine and the like may be utilized to pick up the acid which is liberated during the course of the reaction. In some circumstances the addition of an iodide salt, preferably an alkali metal iodide, e.g. potassium iodide, is appropriate. Somewhat elevated temperatures and stirring may enhance the rate of the reaction.

The compounds of formula (I) wherein $R^1$ is $C_{1-6}$alkyl optionally substituted with aryl; $C_{3-6}$cycloalkyl or a radical of formula (a-3) and the carbon atom of said $R^1$ radical adjacent to the nitrogen atom bearing said $R^1$ contains at least one hydrogen atom, said radicals being represented by $R^{1-a}$, and said compounds by formula (I-a), may also be prepared by the reductive N-alkylation of an intermediate of formula (V) with a ketone or aldehyde of formula $R^{1-b}$=O (VI). In formula (VI), $R^{1-b}$ represents a geminal bivalent radical derived from $R^{1-a}$-H wherein two geminal hydrogen atoms are replaced by =O.

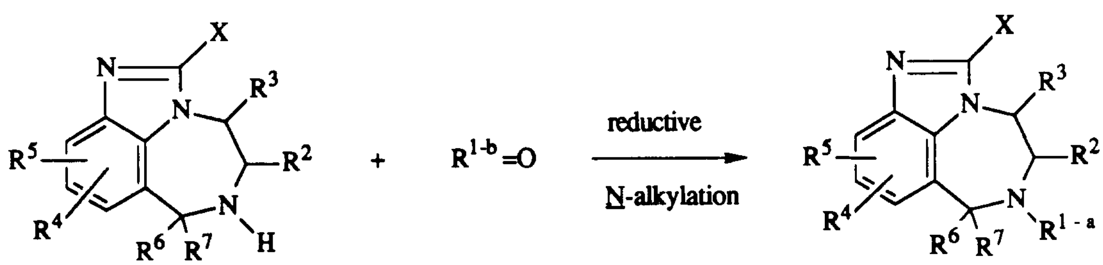


(V) (VI) (I-a)

Said reductive N-alkylation reaction may conveniently be carried out by catalytically hydrogenating the reactants in a suitable reaction-inert organic solvent according to art-known catalytic hydrogenation procedures. The reaction mixture may be stirred and/or heated in order to enhance the reaction rate. Suitable solvents are, for example, water; $C_{1-6}$alkanols, e.g. methanol, ethanol, 2-propanol and the like; ethers, e.g. 1,4-dioxane and the like; halogenated hydrocarbons, e.g. trichloromethane and the like; dipolar aprotic solvents, e.g. N,N-dimethylformamide, dimethyl sulfoxide and the like; esters, e.g. ethyl acetate and the like; or a mixture of such solvents. The term art-known catalytic hydrogenation procedures means that

the reaction is carried out under a hydrogen atmosphere and in the presence of an appropriate catalyst such as, for example, palladium-on-charcoal, platinum-on-charcoal and the like. In order to prevent the undesired further hydrogenation of certain functional groups in the reactants and the reaction products it may be advantageous to add an appropriate catalyst-poison to the reaction mixture, e.g., thiophene and the like. Alternatively, said reductive N-alkylation may also be performed following art-known reduction procedures by treating a stirred and, if desired, heated mixture of the reactants with a reducing agent such as, for example, sodium borohydride, sodium cyanoborohydride, formic acid or a salt thereof, in particular the ammonium salt thereof.

The compounds of formula (I) wherein X is OH, SH or $NHR^{16}$, said compounds being represented by formula (I-b) and said radical X by $X^1$, can generally be prepared by condensing a 9-amino-2,3,4,5-tetrahydro-1H-1,4-benzodiazepine of formula (VII) with a reagent of formula (VIII), wherein $L^1$ is an appropriate leaving group and $X^2$ is =O, =S or $=NR^{16}$.

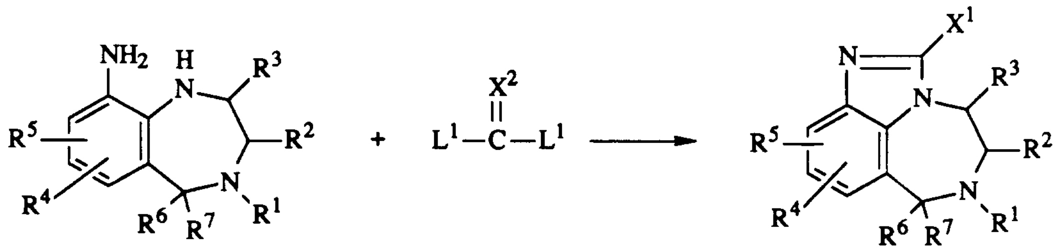

(VII) (VIII) (I-b)

Appropriate agents of formula (VIII) are for example urea, di($C_{1-6}$alkyl)carbonate, carbonoic dichloride, trichloromethyl chloroformate, 1,1'-carbonylbis[1H-imidazole], alkali metal, alkaline earth metal or ammonium isocyanates, phenyl isocyanate, benzoyl isoyocyanate, thiourea, carbonothioic dichloride, carbon disulfide, 1,1'-carbonothioyl-bis[1H-imidazole], xanthogenates, alkali metal, alkaline earth metal or ammonium isothiocyanates, phenyl isothiocyanate, benzoyl isothiocyanate, 1,3-dithiolane-2-thione, a guanidine salt, e.g., guanidine carbonate, hydrochloride, nitrate and the like salts of guanidine, N-cyanoguanidine, N-cyanodiphenoxymethanimine and the like. Said condensation reaction may conveniently be conducted by stirring and optionally heating the reactants in a reaction-inert solvent, such as, for example, an aromatic hydrocarbon, e.g. benzene, methylbenzene, dimethylbenzene and the like; a halogenated hydrocarbon, e.g. trichloromethane, tetrachloromethane, chlorobenzene and the like; an ether, e.g. tetrahydrofuran, 1,4-dioxane, 1,1'-oxybisbutane, 1,1'-oxybis(2-methoxyethane), 1,2-bis(2-methoxyethoxy)ethane and the like; a dipolar aprotic solvent, e.g. N,N-dimethylformamide, N,N-dimethylacetamide, dimethyl sulfoxide, 1-methyl-2-pyrrolidinone, pyridine, methylpyridine, dimethylpyridine, tetrahydrothiophene 1,1-dioxide and the like; or a mixture of such solvents. In some instances however, it may be preferable to heat the reactants without a solvent. Further it may be appropriate to add to the reaction mixture a base such as, for example, a tertiary amine, e.g. N,N-diethylethanamine, N-ethyl-N-(1-methylethyl)-2-propanamine, 4-methylmorpholine and the like amines. When said reagent of formula (VIII) is carbon disulfide, the reaction can also be conducted conveniently in an alkanol such as, for example, methanol, ethanol, propanol and the like, in the presence of a base such as sodium or potassium hydroxide and the like or in carbon disulfide as solvent and in the presence of a suitable base such as, for example, an alkyl magnesium halide, e.g. ethyl magnesium bromide, an alkyl lithium, e.g. butyllithium, an amine, e.g., N,N-diethylethanamine, a carbodiimide, e.g. N,N-dicyclohexylcarbodiimide and the like reagents. Or, alternatively the latter reaction may also be conducted in basic solvent such as, for example, pyridine and the like, in the presence of a phosphite such as, for example, diphenylphosphite.

The compounds of formula (I-b) wherein $X^1$ is SH, said compounds being represented herebelow in their equivalent tautomeric form in formula (I-b-2), can be prepared by thionation of the compounds of formula (I-b) wherein $X^1$ is OH, said compounds represented by formula (I-b-1), with 2,4-bis(4-methoxyphenyl)-1,3-dithia-2,4-diphosphetane-2,4-disulfide (Lawesson's reagent) in an appropriate reaction-inert solvent. Such solvents are for example, aromatic hydrocarbons, e.g. benzene, methylbenzene, dimethylbenzene, dipolar aprotic solvents, e.g. hexamethylphosphoric triamide (HMPA) and the like solvents.

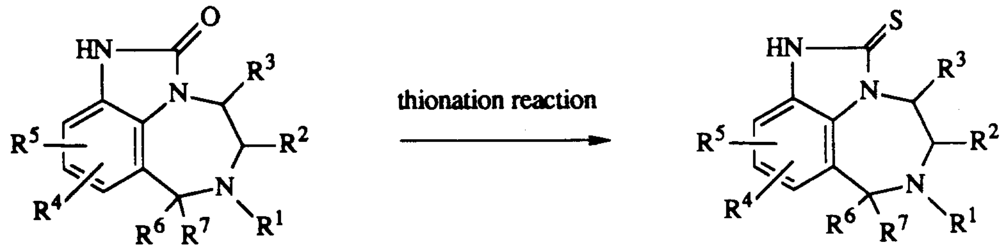


(I-b-1) (I-b-2)

Alternatively, the compounds of formula (I-b-2) may also be obtained by thionation of the compounds of formula (I-b-1) with phosphorus pentasulfide.

The compounds of formula (I-b-2) may also be obtained by direct thiation of a tetrahydroimidazo[4,5,1-jk][1,4]benzodiazepine of formula (IX) with elemental sulfur at an elevated temperature.

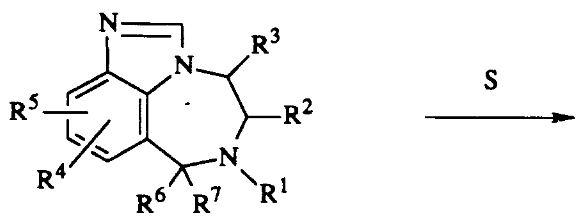


(I-b-2)

(IX)

Said reaction may conveniently be conducted without a solvent at a temperature above 200 °C, more particularly a temperature in the range of 230 to 250 °C.

The compounds of formula (I-b-2) may also be prepared by the combined reduction-thiocarbonylation of a 9-nitrobenzodiazepine of formula (X) in the presence of an alkali metal sulfide or hydrogen sulfide, and carbon disulfide.

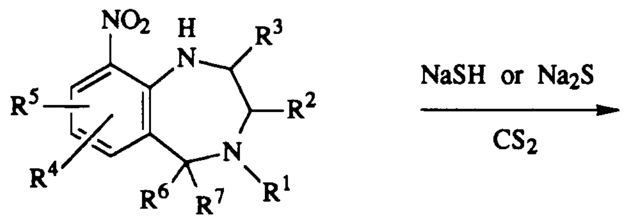


(I-b-2)

(X)

Said reduction-thiocarbonylation reaction may conveniently be conducted by stirring the reactants in a reaction-inert solvent, optionally at an elevated temperature.

The compounds of formula (I) may also be prepared by cycling a benzimidazole of formula (XI) in a suitable reaction-inert solvent, optionally in the presence of a base and optionally at an elevated temperature.

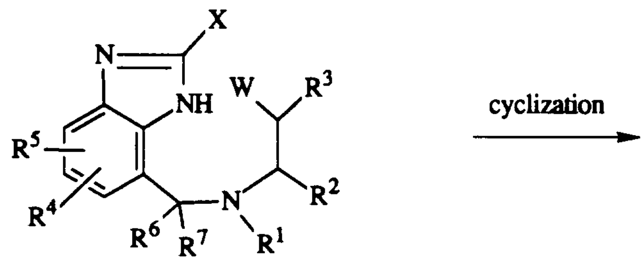


(I)

(XI)

In formula (XI), W represents a reactive leaving group as defined hereinbefore. Said cyclization reaction may conveniently be conducted by stirring, and, if desired, heating the starting material. Suitable solvents are, for example, aromatic hydrocarbons, e.g. benzene, methylbenzene, dimethylbenzene and the like, halogenated hydrocarbons, e.g. trichloromethane, tetrachloromethane, chlororbenzene and the like, ethers, e.g. tetrahydrofuran, 1,4-dioxane and the like, dipolar aprotic solvents e.g. N,N-dimethylformamide, N,N-dimethylacetamide, acetonitrile, dimethylsulfoxide, pyridine and the like. Bases which may conveniently be employed in said cyclization reaction are, for example, alkali metal or alkaline earth metal carbonates, hydrogen carbonates, hydroxides, oxides, amides, hydrides and the like. In some instances the addition to the reaction mixture of a iodide salt, preferably an alkali metal iodide, e.g. potassium iodide, may be advantageous.

The compounds of formula (I) may also be converted into each other following art-known functional group transformation reactions. For example, the compounds wherein $R^{16}$ and/or $R^{17}$ are $C_{1-6}$alkyl may be prepared by N-alkylating the compounds of formula (I) wherein $R^{16}$ and/or $R^{17}$ are hydrogen with a reagent ($C_{1-6}$alkyl)-W, wherein W is a leaving group as defined hereinabove. The compounds wherein $R^{16}$ is $C_{1-6}$alkylcarbonyl or $C_{1-6}$alkyloxycarbonyl may be prepared by N-acylating the compounds wherein $R^{16}$ is hydrogen with an appropriate acyl halide, e.g., acetyl chloride, propanoyl chloride and the like, a carboxylic acid anhydride, e.g., acetic anhydride, propanoic anhydride and the like, a $C_{1-6}$alkylcarbonochloridate, e.g., 1,1-dimethylethylethyl carbonochloridate and the like acylating reagents. In a similar way the compounds wherein $R^{16}$ is ($C_{1-6}$alkyl or aryl)sulfonyl may be prepared by N-sulfonylating the compounds wherein $R^{16}$ is hydrogen with an appropriate ($C_{1-6}$alkyl or aryl)sulfonyl halide.

In all of the foregoing and in the following preparations, the reaction products may be isolated from the reaction mixture and, if necessary, further purified according to methodologies generally known in the art.

A number of intermediates and starting materials in the foregoing preparations are known compounds which may be prepared according to art-known methodologies of preparing said or similar compounds. Most intermediates however, are new and are especially developed for the preparation of the compounds of formula (I). A number of preparation methods, in particular for said novel intermediates, is described hereinafter in more detail.

The intermediates of formula (II) can generally be prepared from the compounds of formula (I-b-1) by reaction with a halogenating reagent such as, for example, phosphoryl chloride, phosphorous trichloride, phosphorous tribromide, thionyl chloride, oxalyl chloride and the like reagents, optionally at a elevated temperature, in particular the reflux temperature of the reaction mixture, and optionally in the presence of a base such as, for example, sodium carbonate, sodium hydrogen carbonate, potassium carbonate and the like. The reaction can be conducted in an excess of the halogenating reagent as solvent, optionally in admixture with a reaction-inert solvent such as, for example, an aromatic hydrocarbon or an ether.

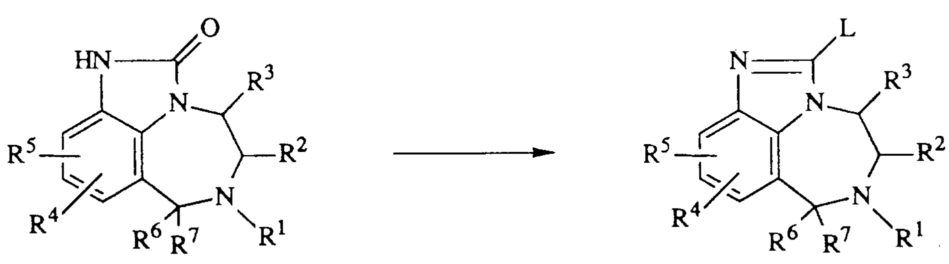

(I-b-1) (II)

The intermediates of formula (V) wherein $R^2$, $R^3$, $R^4$, $R^5$, $R^6$, $R^7$ and X are as defined under formula (I) are novel and can be prepared from benzodiazepin-7-ones of formula (XII) as shown in the next reaction scheme.

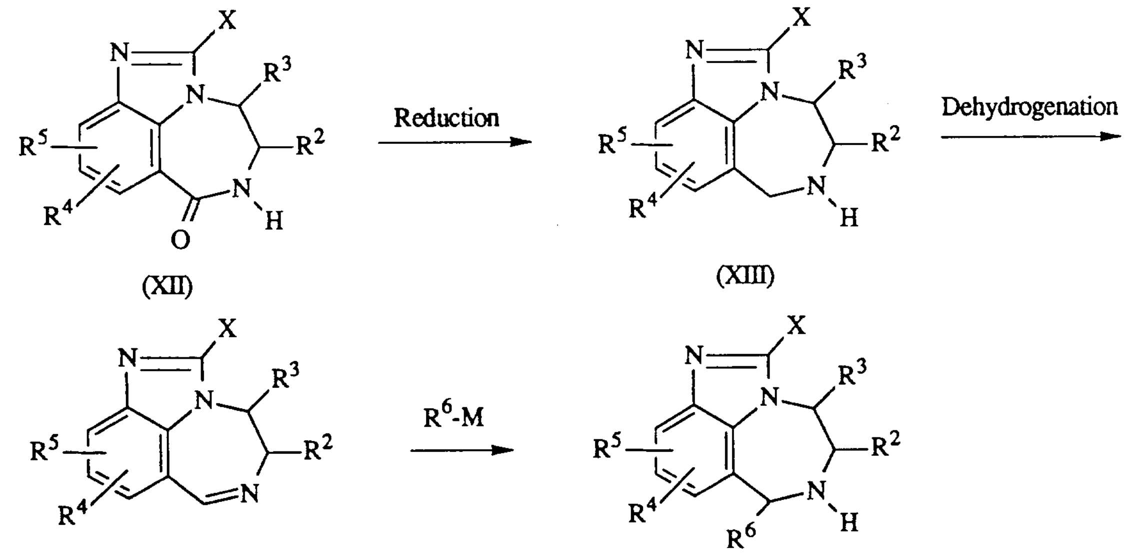


(XII) (XIII)

(XIV) (V-a)

The benzodiazepin-7-ones of formula (XII) can be reduced to benzodiazepines of formula (XIII) with a complex metal hydride, e.g. lithium aluminum hydride and the like in a suitable reaction-inert solvent such as, for example, 1,2-dimethoxyethane, 1,1'-oxy-bis(2-methoxyethane), 2,5,8,11-tetraoxadodecane, methoxybenzene and the like solvents. In order to enhance the rate of said reduction reaction, it may be advantageous to employ an excess of the reducing reagent and to conduct said reaction at an enhanced temperature, in particular the reflux temperature of the reaction mixture.

The thus obtained benzodiazepines of formula (XIII) can be dehydrogenated to intermediates of formula (XIV). Said dehydrogenation can be carried out by oxidation of (XIII) with permanganate or with manganese-(IV)oxide. Said dehydrogenation reaction can be carried out in a suitable reaction-inert solvent such as, for example, water, an alcohol, e.g. methanol, ethanol and the like, an ether, e.g. 1,1'-oxybisethane, tetrahydrofuran and the like or a mixture of such solvents.

Or alternatively, the imine (XIV) may be obtained by reaction with nickel, platinum or chromium catalysts or in the presence of easily reducible substances such as sulfur, amyldisulfide, selenium or sodium amide in liquid ammonia.

The benzodiazepines of formula (V) wherein $R^7$ is hydrogen, said intermediates being represented by formula (V-a), can be prepared from intermediates of formula (XIV) by reaction with organometallic compounds of formula $R^6$-M, wherein M represents a metal group such as, for example, lithium, halo magnesium, copper lithium and the like, in a reaction-inert solvent such as, for example, an ether, e.g. tetrahydrofuran, 1,1'-oxybisethane, 1,2-dimethoxyethane and the like; a hydrocarbon, e.g. hexane, benzene, methylbenzene and the like, or a mixture thereof.

The intermediates of formula (V) can also be obtained from a benzylated compound of formula (I-c) following art-known hydrogenolysis procedures.

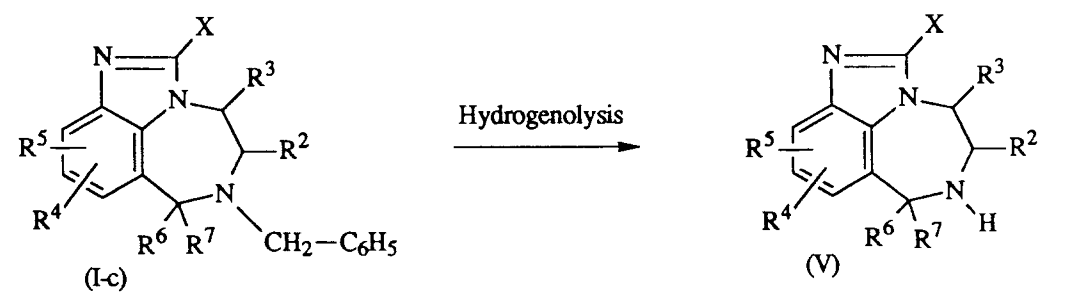


(I-c) (V)

Said debenzylation reaction can be accomplished by stirring a compound of formula (I-c) in an appropriate reaction-inert solvent in the presence of a suitable metal catalyst and under a hydrogen atmosphere. Appropriate solvents are, for example, alkanols, e.g. methanol, ethanol and the like; carboxylic esters, e.g. ethyl acetate; carboxylic acids, e.g. acetic acid, propanoic acid and the like. As examples of suitable metal catalysts there may be mentioned palladium-on-charcoal, platinum-on-charcoal and the like catalysts. In order to prevent the further hydrogenation of the starting material and/or the reaction product it may be appropriate to add a catalyst-poison to the reaction mixture such as, for example thiophene.

The intermediates of formula (V), wherein X is OH, SH or $NHR^{16}$ said intermediates being represented by formula (V-c) and said radical X by $X^1$, can be prepared from intermediates of formula (VII-a) following the condensation reaction with a reagent of formula $L^1$-C(=$X^2$)-$L^1$ (VIII) as described hereinbefore for the preparation of the compounds of formula (I-b) from the intermediates of formula (VII).

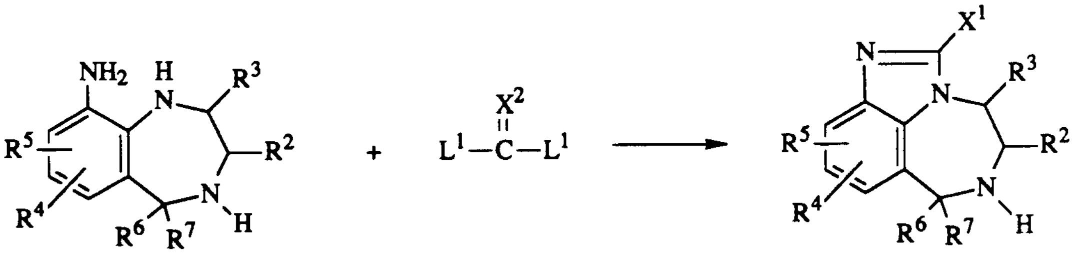

(VII-a) (VIII) (V-c)

The intermediates of formula (V) wherein X is SH, said intermediates being represented by formula (V-c-2), may also be prepared by thionation of intermediates of formula (V-c-1) following the procedures described hereinabove for the preparation of the compounds of formula (I-b-2) from the compounds of formula (I-b-1).

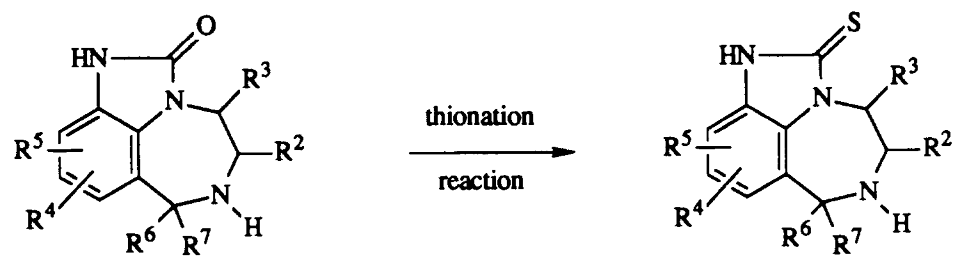


(V-c-1) (V-c-2)

The intermediates of formula (VII) can generally be prepared from a 9-aminobenzodiazepine of formula (VII-a) following N-alkylation reaction procedures such as described hereinabove for the preparation of the compounds of formula (I) and (I-a) from an intermediate of formula (V) with an alkylating reagent (IV) or with an aldehyde or ketone of formula (VI) as defined hereinabove.

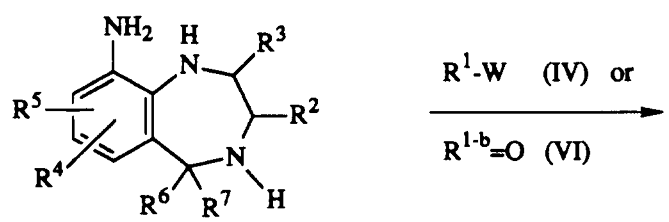


(VII)

(VII-a)

In order to simplify the following reaction schemes, the N-alkylated intermediates wherein $R^1$ is as defined under formula (I) and the $N^4$-unsubstituted intermediates (wherein $R^1$ is replaced by hydrogen) will

be represented hereinafter by formulae wherein $N^4$ is substituted with $R^{1H}$, said $R^{1H}$ representing $R^1$ as defined in formula (I) and hydrogen. In intermediates (XV), (XVI), (XVIII), (XIX), (XXI) and (XXII) of scheme 1 hereinbelow, $R^{1H}$ also defines $C_{1-5}$alkylcarbonyl, aryl$C_{1-5}$alkylcarbonyl or a radical of formula

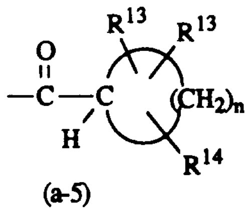

(a-5)

or

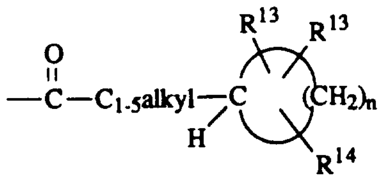

(a-6)

The latter amide intermediates can conveniently be prepared following art-known N-acylation procedures from corresponding intermediates wherein $R^{1H}$ is hydrogen and can be reduced to the corresponding N-alkylated intermediates with complex metal hydrides or hydrides as described under reaction step A of scheme 1 hereinafter. In all of the following reaction schemes, the intermediates wherein $R^{1H}$ is hydrogen can generally also be converted into intermediates wherein $R^{1H}$ is $R^1$ following the above described N-alkylation procedures with and alkylating reagent of formula $R^1$-W (IV) or with an aldehyde or ketone of formula $R^{1-b}=O$ (VI).

The intermediates of formula (VII-H) wherein $R^{1H}$ represents hydrogen or a radical $R^1$ as defined under formula (I), said intermediates representing the intermediates of formula (VII) and (VII-a) wherein the radicals $R^2$, $R^3$, $R^4$, $R^5$, $R^6$ and $R^7$ are as defined under formula (I), are novel and can generally be prepared following the reaction steps shown in the reaction scheme 1 hereinafter.

Scheme 1

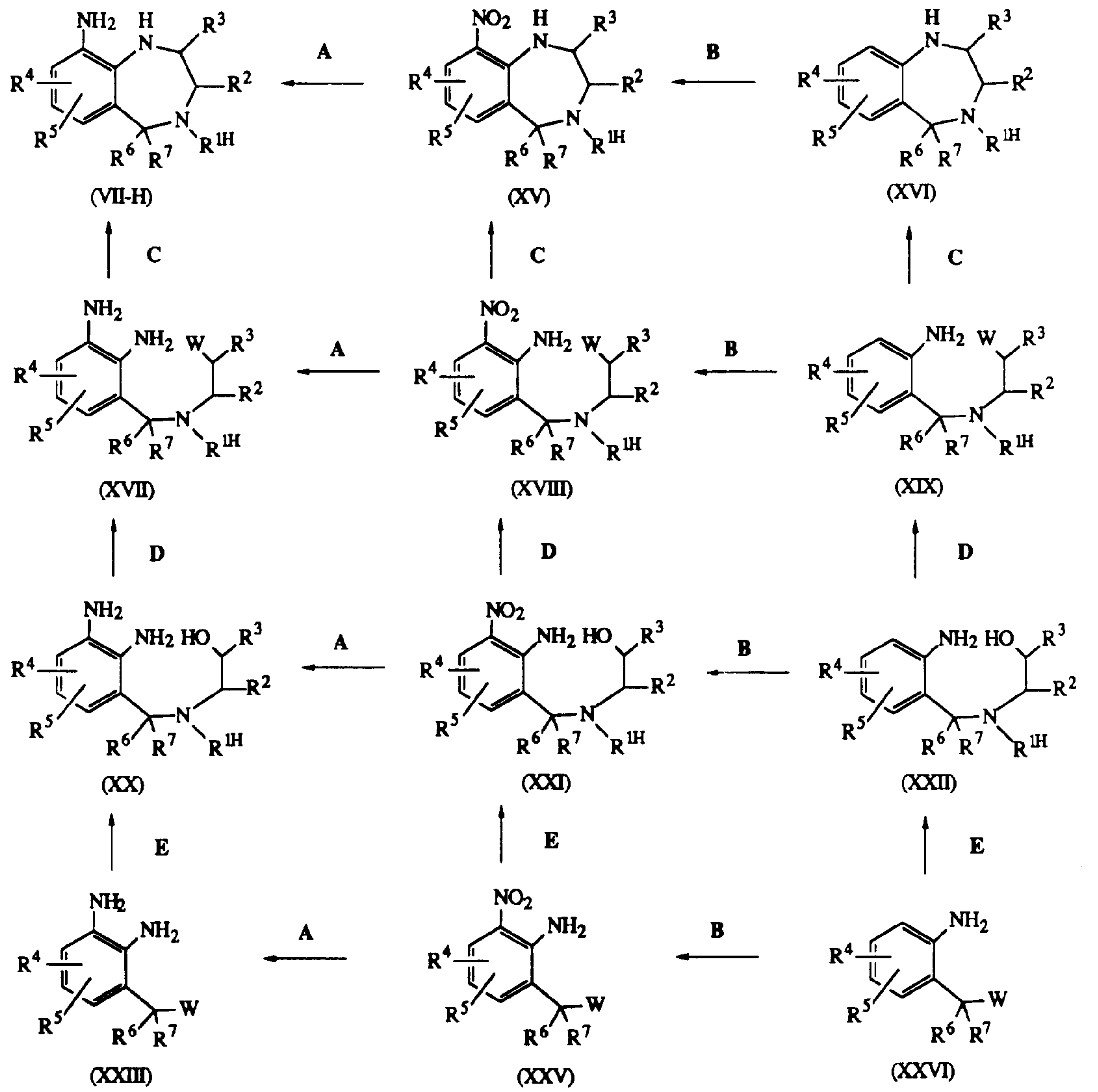


(VII-H) (XV) (XVI)

(XVII) (XVIII) (XIX)

(XX) (XXI) (XXII)

(XXIII) (XXV) (XXVI)

- **A** : nitro-to-amine reduction (if $R^{1H}$ is acyl; also amide-to-amine reduction)
- **B** : nitration
- **C** : cyclization
- **D** : -OH-to-W activation
- **E** : N-alkylation : $R^{1H}NH-CH(R^2)-CH(R^3)OH$ (XXIV)

The aniline derivatives in the reaction scheme may conveniently be prepared by reduction of the corresponding nitrobenzene derivatives following art-known nitro-to-amine reduction procedures (reaction step A). Said reduction may conveniently be conducted by treatment of said nitrobenzenes with a reducing agent such as, for example, a complex metal hydride, e.g. lithium aluminum hydride, sodium bis(2-methoxyethoxy)aluminum hydride; a hydride, e.g. diborane, aluminum hydride and the like, in a reaction-inert solvent such as, for example, 1,1'-oxybisethane, tetrahydrofuran, 1,4-dioxane, 1,2-dimethoxyethane and the like, optionally in the presence of a cosolvent such as an aromatic hydrocarbon, e.g. benzene, methylbenzene and the like, and, if desired, at an elevated temperature. Alternatively, said reduction may also be accomplished by treatment of said nitrobenzene derivatives with sodium dithionite, sodium sulfide, sodium hydrogen sulfide, titanium(III) chloride and the like reducing agents in a suitable solvent, in particular water.

Said nitro-to-amine reduction may also be conducted following art-known catalytic hydrogenation procedures. For example, said reduction may be carried out by stirring the reactants under a hydrogen atmosphere and in the presence of an appropriate catalyst such as, for example, palladium-on-charcoal, platinum-on-charcoal, Raney nickel and the like catalysts. Suitable solvents are, for example, water, alkanols, e.g. methanol, ethanol and the like, esters, e.g. ethyl acetate and the like. When Raney nickel is used as reductant, it may be advantageous to add to the reaction mixture an adjuvant, in particular hydrazine. In order to enhance the rate of said reduction reaction it may be advantageous to elevate the temperature and/or the pressure of the reaction mixture. Undesired further hydrogenation of certain functional groups in the reactants and the reaction products may be prevented by the addition of a catalyst poison such as, for example, thiophene and the like, to the reaction mixture.

The nitrobenzene derivatives in the above reaction scheme 1 can be prepared from benzenamine derivatives following art-known nitration procedures (reaction step B). For example, the starting materials may be nitrated by treatment with concentrated or fuming nitric acid in the presence of concentrated sulfuric acid and optionally in the presence of a cosolvent such as, for example, a halogenated hydrocarbon, e.g. dichloromethane, trichloromethane, tetrachloromethane and the like solvents. Alternatively, said nitration can in some instances also be accomplished conveniently by adding the nitrate salt of the starting material to concentrated sulfuric acid.

The benzodiazepine derivatives (VII-H), (XV) and (XVI) may be obtained from the corresponding aniline derivatives (XVII), (XVIII) and (XIX) following the cyclization procedures such as described hereinabove for the preparation of the compounds of formula (I) from intermediates of formula (XI) (Reaction step C). Said aniline derivatives in turn, wherein W is a reactive leaving group as defined hereinbefore, can be prepared from the corresponding alkanols by treatment with a halogenating reagent such as, for example, thionyl chloride, phosphoryl chloride, phosphorous trichloride and the like; or by treatment with a sulfonylating reagent, e.g. methanesulfonyl chloride, 4-methylbenzenesulfonyl chloride and the like (Reaction step D). Said alkanols may be prepared by N-alkylating appropriately substituted benzene derivatives of formulae (XXIII), (XXV) or (XXVI) with an aminoethanol derivative of formula $R^{1H}NH-CH(R^2)-CH(R^3)OH$ (XXIV) following art-known N-alkylation procedures such as described hereinabove (Reaction step E).

The intermediates of formula (XV) wherein $R^{1H}$ and $R^7$ are both hydrogen, said intermediates being represented by (XV-a) can also be obtained by reacting an appropriately substituted nitrobenzene (XXVII) and a diamino reagent of formula (XXVIII). Herein is Y either hydrogen or a removable protective group such as, for example, $C_{1-6}$alkylcarbonyl, e.g. acetyl, trichloroacetyl and the like, a benzyl group, a $C_{1-6}$alkyloxycarbonylgroup, e.g. 1,1-dimethylethyloxycarbonyl, and the like groups commonly used to protect an amino group.

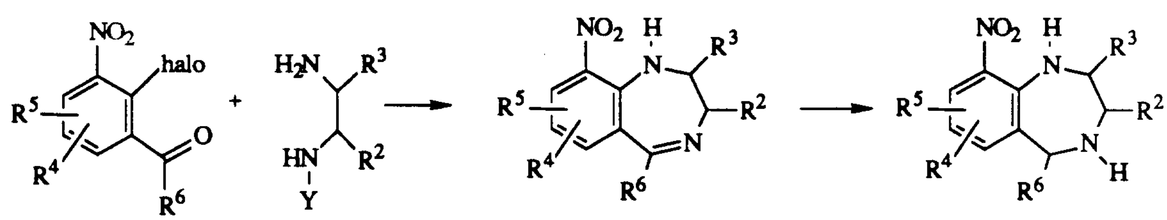

(XXVII) (XXVIII) (XXIX) (XV-a)

Said reaction may conveniently be conducted by condensing the diamino reagent of formula (XXVIII) with the nitrobenzene of formula (XXVII), optionally removing the protective group by alkaline or acid hydrolysis or by catalytic hydrogenation and reducting the thus obtained intermediate (XXIX). Said condensation reaction can conveniently be conducted in a suitable reaction-inert solvent such as, for example, an alkanol, e.g. methanol, ethanol, 2-propanol, 1-butanol and the like; an aromatic hydrocarbon, e.g. benzene, methylbenzene, dimethylbenzene and the like; a halogenated hydrocarbon, e.g. trichloromethane, tetrachloromethane and the like; an ether, e.g. tetrahydofuran, 1,4-dioxane, 1,1'-oxybisbutane, 1,1'-oxy(2-methoxyethane) and the like; a ketone, e.g. 2-propanone, 4-methyl-2-pentanone and the like; a dipolar aprotic solvent, e.g. N,N-dimethylformamide, N,N-dimethylacetamide, dimethyl sulfoxide and the like; or a mixture of such solvents. It may be appropriate to add a base such as an alkali metal or earth alkaline metal carbonate, e.g. sodium carbonate, sodium hydrogen carbonate and the like, to the reaction mixture. Said condensation reaction can conveniently be conducted at an elevated temperature, in particular at the reflux temperature of the reaction mixture. Said reductions in the above procedure may conveniently be conducted by reacting the intermediate imines with a suitable reductive reagent such as, for example,

sodium borohydride, sodium cyanoborohydride and the like reductive reagents.

The preparation of the intermediates of formula (VII-H) wherein $R^3$ is hydrogen, said intermediates being represented by formula (VII-H-$\alpha$) can be prepared following the reaction pathways described in scheme 2 hereinbelow. Reaction steps designated A through D are intended to refer back to the analogous reaction steps described in reaction scheme 1. In all of the following schemes, those compounds wherein $R^3$ is hydrogen, are designated by appending the suffix -$\alpha$ to their numerical reference.

The intermediates of formula (VII-H-$\alpha$) can be prepared by reduction of a benzodiazepinone of formula (XXX) or (XXXI) with a complex metal hydride, e.g. lithium aluminum hydride; a hydride, e.g. diborane, aluminum hydride and the like; in a reaction-inert solvent such as, for example, and ether, e.g. 1,1'-oxybisethane, tetrahydrofuran, 1,4-dioxane, 1,2-dimethoxyethane and the like; optionally in the presence of a co-solvent such as an aromatic hydrocarbon, e.g. benzene, methylbenzene and the like; and optionally at an elevated temperature (reaction steps F and G). Depending upon the severity of the reaction conditions the intermediate (XXXI) may be reduced immediately to (VII-H-$\alpha$) or the intermediate (XXX) may be obtained.

The benzodiazepinones in scheme 2 can be obtained by cyclizing (reaction step H) the corresponding acyclic intermediates of formula (XXXIII), (XXXIV) and (XXXV), wherein R represents a group such as $C_{1-6}$alkyl or aryl,

a) by heating without a solvent under an inert atmosphere, optionally under reduced pressure;

b) by treating with a bifunctional catalyst such as, for example, acetic acid, 2-hydroxypyridine, pyrazole, 1,2,4-triazole and the like, in a reaction-inert solvent such as, for example, an aromatic hydrocarbon, e.g. methylbenzene, dimethylbenzene and the like; optionally at an elevated temperature; or

c) by hydrolyzing the ester and subsequently treating the corresponding carboxylic acid (R = H) with an appropriate acid, such as, for example, a hydrohalic acid, e.g. hydrochloric acid; sulfuric acid, phosphoric acid and the like acids ; or with a halogenating reagent such as, for example, thionyl chloride and the like.

Scheme 2

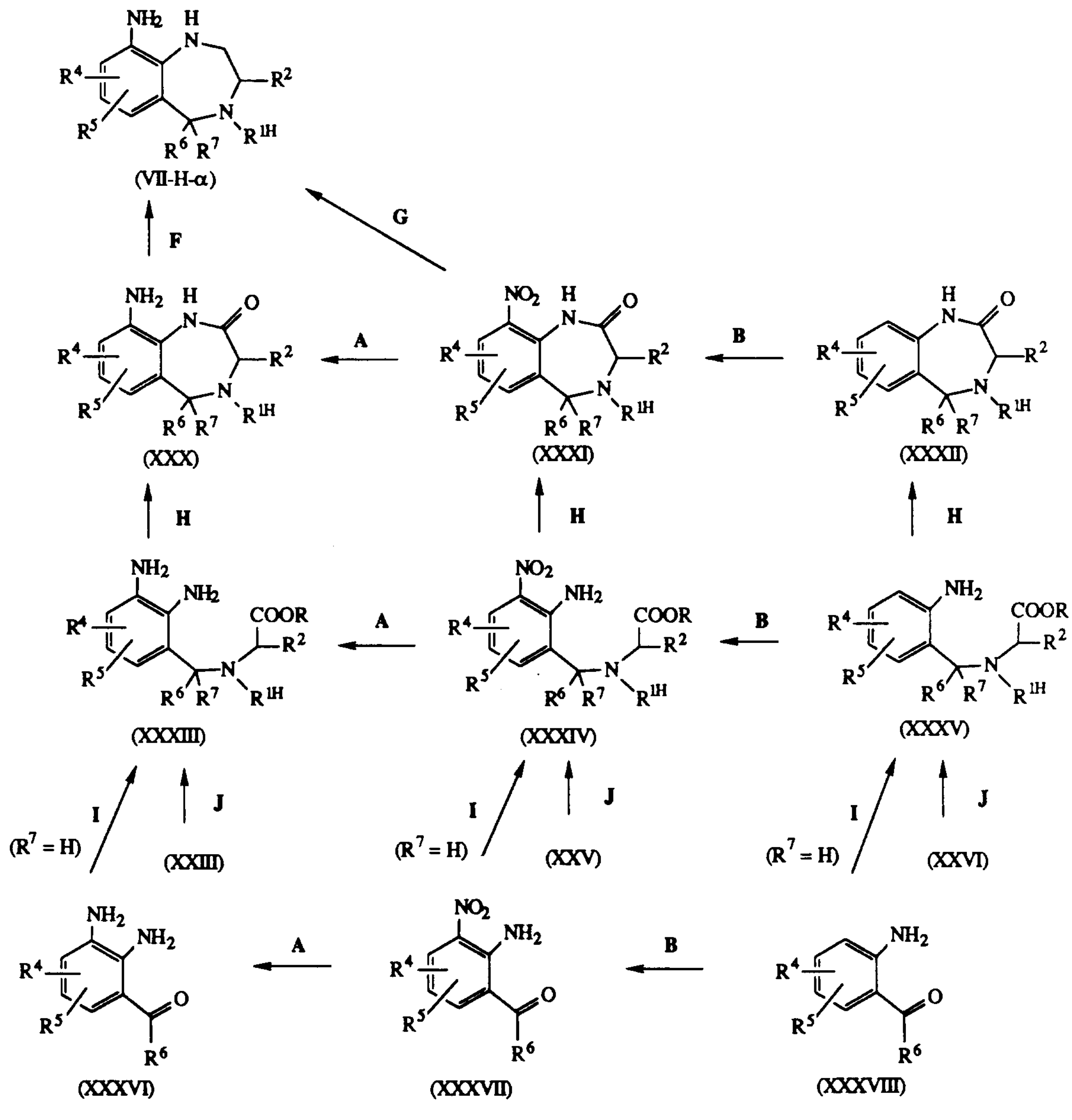


(VII-H-α) (XXX) (XXXI) (XXXII) (XXXIII) (XXXIV) (XXXV) (XXIII) (XXV) (XXVI) (XXXVI) (XXXVII) (XXXVIII)

**F** : amide-to-amine reduction
**G** : (nitro-to-amine) and amide-to-amine reduction
**H** : cyclization to benzodiazepinone
**I** : reductive N-alkylation of $R^{1H}$-NH-CH($R^2$)-COOR (XXXIX)
**J** : N-alkylation of $R^{1H}$-NH-CH($R^2$)-COOR (XXXIX)

The intermediates (XXXIII), (XXXIV) and (XXXV) can be prepared from an appropriately protected amino acid of formula $R^{1H}$-NH-CH($R^2$)-COOR (XXXIX) wherein R is $C_{1-6}$alkyl or aryl, by a reductive N-alkylation reaction or a N-alkylation reaction with an appropriate substituted benzene derivative, by stirring the reactants at reflux temperature in a reaction-inert solvent such as, for example, trichloromethane, pyridine and the like solvents.

The intermediates of formula (XII) and (XIII) wherein X is $X^1$, i.e. OH, SH or $NHR^{16}$, said intermediates being represented by (XII-b) and (XIII-b), may be obtained following the reaction steps shown in reaction scheme 3 below. Reaction steps designated A through D and F are intended to refer back to the analogous reaction steps described in reaction scheme 1.

The intermediates of formula (XII-b) and (XIII-b) can be prepared from a 9-amino-benzodiazepin-5-one of formula (XL), respectively a 9-aminobenzodiazepine (VII-b) by condensation with $L^1$-C(=$X^2$)-$L^1$ (VIII) (reaction step K), as described hereinbefore for the preparation of (I-b) from (VII) and (VIII).

The amide derivatives (XLVI), (XLVII) and (XLVIII) in reaction scheme 3 can conveniently be prepared by N-acylating an ethanolamine of formula (XXIV) wherein $R^{1H}$ is H, said formula being represented by $NH_2$-CH($R^2$)-CH($R^3$)-OH (XXIV-a) with an appropriately substituted 2-aminobenzoic acid derivative of formula (XLIX), (L) or (LI) wherein $L^2$ represents hydroxy or a leaving group such as, for example, halo, e.g. chloro or bromo; alkylcarbonyloxy, e.g. acetyl; alkyloxy, e.g. methoxy, ethoxy and the like; or imidazolyl and the like leaving groups. Said N-acylation reaction (reaction step M) may be carried out by stirring the reactants in a reaction-inert solvent, optionally at an elevated temperature. In those instances where $L^2$ represents hydroxy, said N-acylation reaction may also be carried out by treating the reactants with reagents capable of forming amides such as, for example, N,N-dicyclohexylcarbodiimide (DCC) optionally in the presence of a catalyst such as hydroxybenzotriazole (HOBT) or 4-dimethylaminopyridine (DMAP); 2-chloro-1-methylpyridinium iodide, 1,1'-carbonylbis[1H-imidazole], 1,1'-sulfonylbis[1H-imidazole] and the like reagents. Suitable solvents are halogenated hydrocarbons, e.g. dichloromethane, trichloromethane and the like, ethers, e.g. tetrahydrofuran, 1,4-dioxane and the like, dipolar aprotic solvents, e.g. N,N-dimethylformamide, N,N-dimethylacetamide, pyridine and the like; or mixtures of such solvents.

Scheme 3

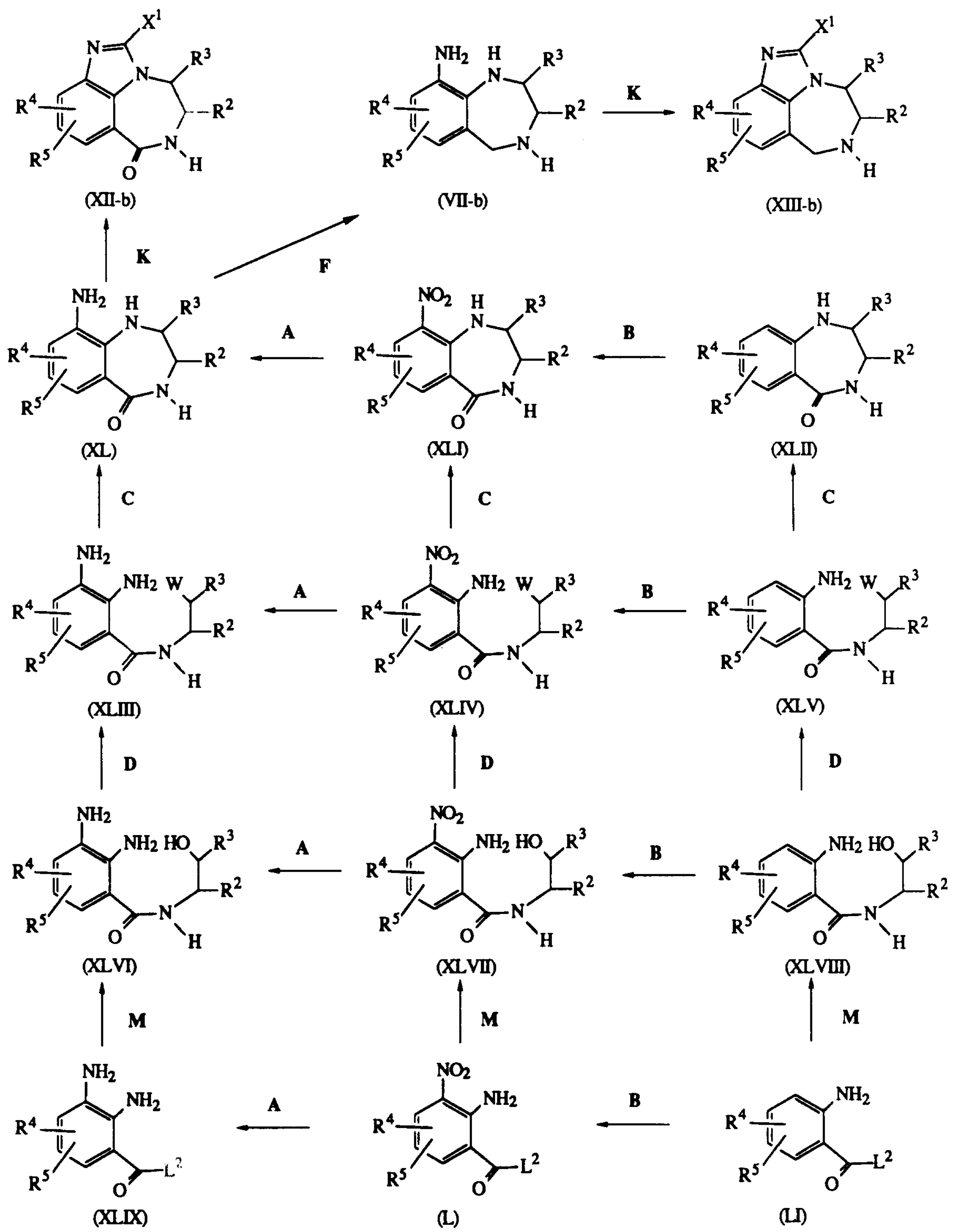


(XII-b) (VII-b) (XIII-b) (XL) (XLI) (XLII) (XLIII) (XLIV) (XLV) (XLVI) (XLVII) (XLVIII) (XLIX) (L) (LI)

**K** : condensation ; $L^1$-C(=$X^2$)-$L^1$ (VIII)

**M** : N-acylation reaction of $H_2N$-CH($R^2$)-CH($R^3$)OH (XXIV-a)

In all of the foregoing reaction schemes, the chemical designation of the intermediates defines the mixture of all possible stereochemically isomeric forms; mixtures of a number of possible stereochemically isomeric forms such as, for example, diastereomeric mixtures, enantiomeric mixtures, e.g. racemates and enriched enantiomeric mixtures; and the enantiomerically pure isomeric forms of the basic molecular

structure.

Stereochemically isomeric forms of the intermediates described in the foregoing reaction schemes and of the compounds of formula (I) may be obtained by the application of art-known procedures. For example, diastereoisomers may be separated by physical separation methods such as destillation, selective crystallization, chromatographic techniques, e.g. counter current distribution, liquid chromatography and the like techniques.

Enantiomerically pure intermediates can conviently be obtained from the enantiomerically pure isomeric forms of the appropriate starting materials, provided that the subsequent reactions occur stereospecifically. Particularly interesting enantiomerically pure starting materials for use in the foregoing reaction schemes are aminoacids and/or substituted derivatives thereof, having the formula $R^{1H}NH-CHR^2-COOR$ (XXXIX), and the corresponding aminoalkanols and/or substituted derivatives thereof, having the formula $R^{1H}NH-CH(R^2)-CH(R^3)OH$ (XXIV) or (XXIV-a) (wherein $R^{1H}$ is hydrogen).

Alternatively, enantiomerically pure intermediates may also be obtained by separating the corresponding racemates for example, by the selective crystallization of their diastereomeric salts with optically active resolving agents, chromatography of diastereomeric derivates, chromatography of the racemate over a chiral stationary phase and the like techniques.

The compounds of formula (I) show antiviral and in particular antiretroviral properties. Until recently, retroviruses were considered to be the pathogenic agents in a number of non-human warm-blooded animal diseases only, unlike viruses which have been known for quite some time to be the cause of a large number of diseases in warm-blooded animals and humans alike. However, since it has been established that a retrovirus, Human Immunodeficiency Virus (HIV), also known as LAV, HTLV-III or ARV, is the etiological agent of Acquired Immune Deficiency Syndrome (AIDS) in humans, retroviral infections and the treatment of subjects suffering therefrom have received the utmost attention. The HIV virus preferentially infects human T-4 cells and destroys them or changes their normal function, particularly the coordination of the immune system. As a result, an infected patient has an everdecreasing number of T-4 cells, which moreover behave abnormally. Hence, the immunological defense system is unable to combat infections and neoplasms and the HIV infected subject usually dies by opportunistic infections such as pneumonia, or by cancers, rather than as a direct result of HIV infections. Other conditions associated with HIV infection include thrombocytopaenia, Kaposi's sarcoma and infection of the central nervous system characterized by progressive demyelination, resulting in dementia and symptoms such as, progressive dysarthria, ataxia and disorientation. HIV infection further has also been associated with peripheral neuropathy, progressive generalized lymphadenopathy (PGL) and AIDS-related complex (ARC). The antiviral, in particular antiretroviral and especially the anti-HIV properties of the compounds of formula (I) suggest said compounds to be useful antiviral chemotherapeutical agents for the prophylaxis or treatment of warm-blooded animals suffering from viral infections, more particularly for the treatment of humans infected by HIV virus.

Due to their antiviral and in particular their antiretroviral properties, the compounds of formula (I), their pharmaceutically acceptable salts and the stereochemically isomeric forms thereof, are useful in the treatment of warm-blooded animals infected with viruses, in particular retroviruses or for the prophylaxis of said warm-blooded animals. In general, the compounds of the present invention may be useful in the treatment of warm-blooded animals infected with viruses whose existence is mediated by, or depends upon, the enzyme reverse transcriptase. Examples of human retroviral infections include HIV and HTLV-I (human T-lymphotropic virus type I), causing leukemia and lymphoma. As an example of non-human animal retroviral infection there may be mentioned FeLV (feline leukemia virus) which causes leukemia and immunodeficiency. Conditions which may be prevented or treated with the compounds of the present invention, especially conditions associated with HIV and other pathogenic retroviruses, include AIDS, AIDS-related complex (ARC), progressive generalized lymphadenopathy (PGL), as well as chronic CNS diseases caused by retroviruses, such as, for example HIV mediated dementia and multiple sclerosis.

In view of their antiviral, in particular antiretroviral activity, the subject compounds may be formulated into various pharmaceutical forms for administration purposes. To prepare the pharmaceutical compositions of this invention, an effective amount of the particular compound, in base or acid addition salt form, as the active ingredient is combined in intimate admixture with a pharmaceutically acceptable carrier, which carrier may take a wide variety of forms depending on the form of preparation desired for administration.These pharmaceutical compositions are desirably in unitary dosage form suitable, preferably, for administration orally, rectally, percutaneously, or by parenteral injection. For example, in preparing the compositions in oral dosage form, any of the usual pharmaceutical media may be employed, such as, for example, water, glycols, oils, alcohols and the like in the case of oral liquid preparations such as suspensions, syrups, elixirs and solutions: or solid carriers such as starches, sugars, kaolin, lubricants, binders, disintegrating agents and the like in the case of powders, pills, capsules and tablets. Because of their ease in administration,

tablets and capsules represent the most advantageous oral dosage unit form, in which case solid pharmaceutical carriers are obviously employed. For parenteral compositions, the carrier will usually comprise sterile water, at least in large part, though other ingredients, for example, to aid solubility, may be included. Injectable solutions, for example, may be prepared in which the carrier comprises saline solution, glucose solution or a mixture of saline and glucose solution. Injectable suspensions may also be prepared in which case appropriate liquid carriers, suspending agents and the like may be employed. In the compositions suitable for percutaneous administration, the carrier optionally comprises a penetration enhancing agent and/or a suitable wetting agent, optionally combined with suitable additives of any nature in minor proportions, which additives do not cause a significant deleterious effect to the skin. Said additives may facilitate the administration to the skin and/or may be helpful for preparing the desired compositions. These compositions may be administered in various ways, e.g., as a transdermal patch, as a spot-on, as an ointment. Acid addition salts of (I) due to their increased water solubility over the corresponding base form, are obviously more suitable in the preparation of aqueous compositions. It is especially advantageous to formulate the aforementioned pharmaceutical compositions in dosage unit form for ease of administration and uniformity of dosage. Dosage unit form as used in the specification and claims herein refers to physically discrete units suitable as unitary dosages, each unit containing a predetermined quantity of active ingredient calculated to produce the desired therapeutic effect in association with the required pharmaceutical carrier. Examples of such dosage unit forms are tablets (including scored or coated tablets), capsules, pills, powder packets, wafers, injectable solutions or suspensions, teaspoonfuls, tablespoonfuls and the like, and segregated multiples thereof.

The present invention is also related with a method of treating viral diseases in warm-blooded animals suffering from said viral diseases by administering an effective antiviral amount of a compound of formula (I), a pharmaceutically acceptable acid addition salt or a stereoisomeric form thereof. Those of skill in the treatment of viral diseases could easily determine the effective antiviral amount from the test results presented herein. In general it is contemplated that an effective amount would be from 0.1 mg/kg to 200 mg/kg body weight, and in particular from 1 mg/kg 50 mg/kg body weight. It may be appropriate to administer the required dose as two, three, four or more sub-doses at appropriate intervals throughout the day. Said sub-doses may be formulated as unit dosage forms, for example, containing 1 to 1000 mg, and in particular 5 to 200 mg of active ingredient per unit dosage form.

The following examples are intended to illustrate and not to limit the invention in all its aspects. Unless otherwise stated, all parts therein are by weight.

## Experimental part

### A. Preparation of the intermediates

#### Example 1

a) To a mixture of 1.99 parts of 1-(2-chloro-3-nitrophenyl)-1-ethanone, 0.84 parts of sodium hydrogen carbonate and 39.5 parts of methanol there were added 0.60 parts of 1,2-ethanediamine under an argon atmosphere. After refluxing for 23 hours and subsequent cooling to room temperature, there were added 1.26 parts of sodium cyanotrihydroborate. The whole was stirred for 7 hours at room temperature and was then neutralized with methanol saturated with hydrochloric acid. Stirring was continued for 15 hours. The mixture was acidified with HCl 3N, stirred for 1/2 hour and concentrated. The residue was partitioned between sodium hydroxide 3N and dichloromethane. The organic layer was dried, filtered and evaporated, yielding 1.85 parts (89.3%) of 2,3,4,5-tetrahydro-5-methyl-9-nitro-1H-1,4-benzodiazepine (interm. 1).

b) To a stirred mixture of 2.10 parts of intermediate 1, 1.60 parts of sodium carbonate and 18.8 parts of N,N-dimethylformamide there were added 1.74 parts of 1-iodopropane under an argon atmosphere. The mixture was heated to 83-89 °C over 1 hour and was maintained at that temperature for 2 hours. It was then evaporated and the residue was partitioned between 1,1'-oxybisethane and water. The organic layer was dried, filtered and evaporated, yielding 2.49 parts (99.9%) of 2,3,4,5-tetrahydro-5-methyl-9-nitro-4-propyl-1H-1,4-benzodiazepine (interm. 2).

c) To a cooled (0 °C) suspension of 1.52 parts of lithium aluminum hydride in 44.5 parts of tetrahydrofuran there was added dropwise a solution of 2.49 parts of intermediate 2 in 35.6 parts of tetrahydrofuran under a nitrogen atmosphere. The mixture was stirred at 0 °C for 10 min, at room temperature for 15 min and at reflux temperature for 1/2 hour. After cooling to 0 °C, there were added 1.52 parts of water, 1.76 parts of NaOH 15% and 4.56 parts of water. The whole was stirred for 2 hours

at room temperature and then filtered. The solid was refluxed in tetrahydrofuran for 5 min and filtered off again. The combined filtrates were evaporated and the residual oil was dissolved in dichloromethane. This solution was dried, filtered and evaporated, yielding 1.86 parts (84.8%) of 2,3,4,5-tetrahydro-5-methyl-4-propyl-1H-1,4-benzodiazepin-9-amine (interm. 3).

Example 2

a) To a stirred suspension of 414 parts of 2-bromo-3-nitrobenzoic acid in 653 parts of methylbenzene there were added 440 parts of thionyl chloride. The whole was refluxed for 6 hours, cooled and left overnight. The reaction mixture was treated with activated charcoal, filtered over diatomaceous earth and evaporated. The residual oil was triturated with 396 parts of hexane (2x). The product was filtered off and washed with hexane, yielding 363 parts (81.7%) of 2-bromo-3-nitrobenzoyl chloride (interm. 4).

b) To a slightly heated mixture of 37.3 parts of intermediate 4 and 142 parts of 1,1'-oxybisethane there was added a solution of 35.0 parts of $C_2H_5O$-Mg-CH$(COOC_2H_5)_2$ in 92.3 parts of 1,1'-oxybisethane under an argon atmosphere. Heating was continued for 1 1/2 hours. Then there was added a solution of 19 parts of concentrated sulfuric acid in 150 parts of water. The organic layer was separated, washed with NaCl (sat.), dried, filtered and evaporated. The residue was refluxed for 6 hours in a mixture of 28 parts of water, 4.41 parts of acetic acid and 9.75 parts of concentrated sulfuric acid. After cooling, the whole was basified with NaOH (3N) and extracted with 1,1'-oxybisethane. The extract was dried, filtered and evaporated, yielding 29.9 parts (86.9%) of 1-(2-bromo-3-nitrophenyl)ethanone (interm. 5).

c) To 29.6 parts of intermediate 5 there were added 12.9 parts of sodium carbonate and 486 parts of 1-butanol under an argon atmosphere. The whole was heated until the solution became homogeneous and then there were added 9.0 parts of 1,2-propane-diamine. After refluxing for 4 hours, the reaction mixture was evaporated. The residue was partitioned between dichloromethane and water. The organic layer was separated, dried, filtered and evaporated. The residue was purified by column chromatography (HPLC ; silica gel ; $CH_3COCH_3$/hexane 20:80). The eluent of the desired fraction was evaporated, yielding 11.4 parts (43.0%) of 2,3-dihydro-3,5-dimethyl-9-nitro-1H-1,4-benzodiazepine (interm. 6).

d) A mixture of 11.35 parts of intermediate 6; 79 parts of methanol and 3.9 parts of sodium cyanotrihydroborate was stirred overnight at room temperature under an argon atmosphere. There were added an additional 0.2 parts of sodium cyanotrihydroborate and some methanol saturated with hydrochloric acid. The reaction mixture was acidified to pH 1 with hydrochloric acid (3N). The solvent was evaporated and the residue was dissolved in dichloromethane. This solution was washed with 10% $K_2CO_3$ (aq.), dried, filtered and evaporated. The residue was purified by column chromatography (HPLC; silica gel; $CH_3COCH_3$/hexane 1:1). The eluent of the desired fraction was evaporated, yielding 2.3 parts (20.1%) of cis-2,3,4,5-tetrahydro-3,5-dimethyl-9-nitro-1H-1,4-benzodiazepine; mp. 62.0 °C (interm. 7).

e) To 2.18 parts of intermediate 7 there were added successively 1.6 parts of sodium carbonate, 1.64 parts of potassium iodide, 23.5 parts of N,N-dimethylformamide and a solution of 1.81 parts of 1-bromo-3-methyl-2-butene in 23.5 parts of N,N-dimethylformamide. After stirring overnight at room temperature, the reaction mixture was evaporated. The residue was partitioned between dichloromethane and a diluted potassium carbonate solution. The organic layer was separated, dried, filtered and evaporated. The residue was converted into the (E)-2-butenedioate (2:1) salt in 2-propanol. The product was filtered off and dried, yielding 2.82 parts (82.4%) of cis-2,3,4,5-tetrahydro-3,5-dimethyl-4-(3-methyl-2-butenyl)-9-nitro-1H-1,4-benzodiazepine (E)-2-butenedioate (2:1); mp. 128.0 °C (interm. 8).

f) To a cooled (ice-bath) mixture of 1.73 parts of lithium aluminum hydride and 44.5 parts of tetrahydrofuran there was added slowly a solution of 3.28 parts of intermediate 8 in 35.6 parts of tetrahydrofuran under an argon atmosphere. The whole was stirred at 0 °C for 1/2 hour, at room temperature for 3 hours and at reflux temperature for 7 hours. After cooling, there were added slowly 1.7 parts of water, 1.7 ml of NaOH (3N), 5.1 parts of water and 89 parts of tetrahydrofuran. The mixture was filtered and the precipitate was washed with 178 parts of hot tetrahydrofuran. The combined filtrates were evaporated, yielding 2.82 parts (96.2%) of cis-2,3,4,5-tetrahydro-3,5-dimethyl-4-(3-methyl-2-butenyl)-1H-1,4-benzodiazepin-9-amine (interm. 9).

In a similar manner there were also prepared :
2,3,4,5-tetrahydro-5-methyl-4-(3-methyl-2-butenyl)-1H-1,4-benzodiazepin-9-amine (interm. 10),
trans-2,3,4,5-tetrahydro-3,5-dimethyl-4-(3-methyl-2-butenyl)-1H-1,4-benzodiazepin-9-amine (interm. 11),
(2,5-trans)-2,3,4,5-tetrahydro-2,5-dimethyl-4-(3-methyl-2-butenyl)-1H-1,4-benzodiazepin-9-amine (interm. 12),
(2,5-cis)-2,3,4,5-tetrahydro-2,5-dimethyl-4-(3-methyl-2-butenyl)-1H-1,4-benzodiazepin-9-amine (interm. 13).

Example 3

To a solution of 1.34 parts of 7-chloro-2,3,4,5-tetrahydro-5-methyl-4-(3-methyl-2-butenyl)-9-nitro-1H-1,4-benzodiazepine (prepared in a similar manner as intermediate 8) in methanol there were added 0.49 parts of Raney nickel. To the resulting suspension there was added dropwise a solution of 1.09 parts of hydrazine in a small amount of methanol, at reflux temperature and under an argon atmosphere. Refluxing was continued for 1 1/2 hour. After cooling, the catalyst was filtered off and the filtrate was evaporated, yielding 1.3 parts (100%) of 7-chloro-2,3,4,5-tetrahydro-5-methyl-4-(3-methyl-2-butenyl)-1H-1,4-benzodiazepin-9-amine (interm. 14).

Example 4

To a cooled (0 ° C) suspension of 1.77 parts of lithium aluminum hydride in 40.1 parts of tetrahydrofuran there was added dropwise a solution of 1.55 parts of intermediate 1 in 44.5 parts of tetrahydrofuran. The mixture was stirred for 20 min at room temperature and for 1 hour at reflux temperature. After cooling to 0 ° C, there were added 1.8 parts of water in 5.34 parts of tetrahydrofuran, 2.09 parts of NaOH 15% and 5.4 parts of water. The whole was stirred for 1 hour and then filtered. The solid was refluxed in tetrahydrofuran for 5 min and filtered off again. The combined filtrates were dried, filtered and evaporated. The residual oil was dissolved in 120 parts of dichloromethane and this solution was dried, filtered and combined with 2.36 parts of 4-methylmorpholine. The whole was added dropwise to a solution of 1.54 parts of trichloromethyl chloroformate in 120 parts of dichloromethane at 0 ° C. The mixture was concentrated and, after the addition of 50 parts of a mixture of water and 1,4-dioxane (85:15), heated on a steam-bath for 1 hour under argon. It was cooled to room temperature, basified with $NH_4OH$ and extracted with dichloromethane. The extract was dried, filtered and evaporated. The residue was purified by flash column chromatography (silica gel ; $CH_2Cl_2/CH_3OH$ 8:1). The eluent of the desired traction was evaporated and the residue was crystallized from acetonitrile, yielding 0.058 parts (3.66%) of 4,5,6,7-tetrahydro-7-methylimidazo[4,5,1-jk][1,4]-benzodiazepin-2(1H)-one (interm. 15); mp. 158.7 ° C.

B. Preparation of the final compounds

Example 5

To a cooled (0 ° C) mixture of 0.93 parts of intermediate 3; 0.86 parts of 4-methylmorpholine and 40 parts of dichloromethane there was added dropwise a solution of 0.43 parts of trichloromethyl chloroformate in 20 parts of dichloromethane under an argon atmosphere. After string for 1/2 hour at 0 ° C, the product was extracted with a sodium hydrogen carbonate solution. The extract was dried, filtered and evaporated. The residue was purified by flash column chromatography (silica gel ; $CH_2Cl_2/CH_3OH$ 15:1). The eluent of the desired traction was evaporated and the residue was triturated with acetonitrile, yielding 0.32 parts (61.5%) of 4,5,6,7-tetrahydro-7-methyl-6-propylimidazo[4,5,1-jk][1,4]benzodiazepin-2(1H)-one (compound 1); mp. 124.0 ° C.

Example 6

To a solution of 0.93 parts of intermediate 3; 3.95 parts of ethanol and 1 part of water there were added 0.32 parts of potassium hydroxide and, after 8 min, 0.43 parts of carbon disulfide. The mixture was sturdy for 10 min at room temperature and heated for 1 hour at 90 ° C. After cooling to room temperature, there were added 5.6 parts of water and 0.49 parts of acetic acid. The solid was filtered off and partitioned between diluted ammonium hydroxide and dichloromethane. The organic layer was dried, filtered and evaporated. The residue was triturated in acetonitrile and recrystallized from ethanol, yielding 0.28 parts (25.2%) of 4,5,6,7-tetrahydro-7-methyl-6-propylimidazo[4,5,1-jk] [1,4]benzodiazepin-2(1H)-thione (compound 2); mp. 179.1 ° C.

Example 7

A mixture of 2.8 parts of intermediate 9; 2.55 parts of 1,1'-carbonothioylbis[1H-imidazole] and 44.5 parts of tetrahydrofuran was refluxed on a steam bath for 1/2 hour under an argon atmosphere. The reaction mixture was evaporated and the residue was partitioned between dichloromethane and water. The organic layer was separated, dried, filtered and evaporated. The residue was purified by flash column chromatog-

raphy (silica gel ; $CH_2Cl_2/CH_3OH$ 99:1). The eluent of the desired fraction was evaporated and the residue was crystallized from ethanol. The product was filtered off and dried, yielding 1.08 parts (33.2%) of cis-4,5,6,7-tetrahydro-5,7-dimethyl-6-(3-methyl-2-butenyl)imidazo[4,5,1-jk][1,4]benzodiazepine-2(1H)thione; mp. 138.3 ° C (comp. 5).

Example 8

A solution of 0.71 parts of intermediate 14; 0.45 parts of 1,1'-carbonylbis-[1H-imidazole] and 22.3 parts of tetrahydrofuran was stirred for 1 1/2 hour at reflux temperature and overnight at room temperature. The reaction mixture was evaporated and the residue was dissolved in ethyl acetate. This solution was washed successively with water (2x), diluted acetic acid, water (2x) and NaCl (sat.) and was then dried, filtered and evaporated. The residue was purified by flash column chromatography (silica gel; hexane/$CH_3COOC_2H_5$ 3:1). The eluent of the desired fraction was evaporated and the residue was crystallized from acetonitrile. The product was filtered off and dried, yielding 0.36 parts (47.1%) of 9-chloro-4,5,6,7-tetrahydro-7-methyl-6-(3-methyl-2-butenyl)imidazo[4,5,1-jk][1,4]benzodiazepin-2(1H)-one; mp. 138.7 ° C (comp. 9).

All other compounds listed in Table I were prepared following the procedure of the example referred to in the column Ex. No.

Table 1

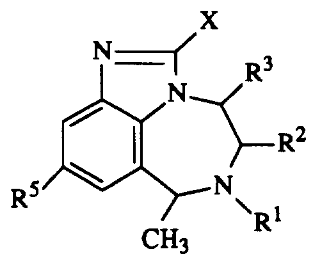

| Co. No. | Ex. No. | X | $R^1$ | $R^2$ | $R^3$ | $R^5$ | physical data |
|---|---|---|---|---|---|---|---|
| 1 | 5 | OH | $-(CH_2)_2-CH_3$ | H | H | H | mp. 124°C |
| 2 | 6 | SH | $-(CH_2)_2-CH_3$ | H | H | H | mp. 179.1°C |
| 3 | 6 | SH | $-CH_2-CH=C(CH_3)_2$ | H | H | H | mp. 192.4°C |
| 4 | 5 | OH | $-CH_2-CH=C(CH_3)_2$ | H | H | H | mp. 108.8°C |
| 5 | 7 | SH | $-CH_2-CH=C(CH_3)_2$ | $CH_3$ | H | H | mp. 138.3°C / cis |
| 6 | 7 | SH | $-CH_2-CH=C(CH_3)_2$ | $CH_3$ | H | H | mp. 138.8°C / trans |
| 7 | 7 | SH | $-CH_2-CH=C(CH_3)_2$ | H | $CH_3$ | H | mp. 191.4°C / trans |
| 8 | 7 | SH | $-CH_2-CH=C(CH_3)_2$ | H | $CH_3$ | H | mp. 185.2°C / cis |
| 9 | 8 | OH | $-CH_2-CH=C(CH_3)_2$ | H | H | Cl | mp. 138.7°C |
| 10 | 7 | SH | $-CH_2-CH=C(CH_3)_2$ | H | H | Cl | mp. 197.4°C |
| 11 | 8 | OH | $-CH_2-CH=C(CH_3)_2$ | $CH_3$ | H | H | cis |
| 12 | 8 | OH | $-CH_2-CH=C(CH_3)_2$ | $CH_3$ | H | H | trans |
| 13 | 5 | $NHCH_3$ | $-CH_2-CH=C(CH_3)_2$ | $CH_3$ | H | H | |
| 14 | 5 | NHOH | $-CH_2-CH=C(CH_3)_2$ | $CH_3$ | H | H | |

C. Pharmacological example

Example 9

A rapid, sensitive and automated assay procedure was used for the in-vitro evaluation of anti-HIV agents. An HIV-1 transformed T4-cell line, MT-4, which was previously shown (Koyanagi et al., Int. J. Cancer, 36, 445-451, 1985) to be highly susceptible to and permissive for HIV infection, served as the target cell line. Inhibition of the HIV-induced cytopathic effect was used as the end point. The viability of both HIV- and mock-infected cells was assessed spectrophotometrically via the in-situ reduction of 3-(4,5-dimethylthiazol-2-yl)-2,5-diphenyltetrazolium bromide (MTT). The 50% cytotoxic dose ($CD_{50}$ in µg/ml) was defined as the concentration of compound that reduced the absorbance of the mock-infected control sample by 50%. The percent protection achieved by the compound in HIV-infected cells was calculated by the following formula:

$$\frac{(OD_T)_{HIV} - (OD_C)_{HIV}}{(OD_C)_{MOCK} - (OD_C)_{HIV}} \quad \text{expressed in \%,}$$

whereby $(OD_T)_{HIV}$ is the optical density measured with a given concentration of the test compound in HIV-infected cells; $(OD_C)_{HIV}$ is the optical density measured for the control untreated HIV-infected cells; $(OD_C)_{MOCK}$ is the optical density measured for the control untreated mock-infected cells; all optical density values were determined at 540 nm. The dose achieving 50% protection according to the above formula was defined as the 50% effective dose ($ED_{50}$ in µg/ml). The ratio of $CD_{50}$ to $ED_{50}$ was defined as the selectivity index (SI).

Table 2

| 50% cytotoxic ($CD_{50}$), 50% effective dose ($ED_{50}$) and selectivity index (SI). | | | |
|---|---|---|---|
| Comp. No. | $CD_{50}$ (µg/ml) | $ED_{50}$ (µg/ml) | SI |
| 2 | 129 | 0.5 | 258 |
| 3 | 177 | 0.1 | 1770 |
| 5 | 23 | 0.24 | 96 |
| 6 | 23 | 0.0048 | 4838 |

D. Composition Examples

Example 10: ORAL DROPS

500 g of the A.I. was dissolved in 0.5 l of 2-hydroxypropanoic acid and 1.5 l of the polyethylene glycol at 60~80 °C. After cooling to 30~40 °C there were added 35 l of polyethylene glycol and the mixture was stirred well. Then there was added a solution of 1750 g of sodium saccharin in 2.5 l of purified water and while stirring there were added 2.5 l of cocoa flavor and polyethylene glycol q.s. to a volume of 50 l, providing an oral drop solution comprising 10 mg/ml of A.I.. The resulting solution was filled into suitable containers.

Example 11 : ORAL SOLUTION

9 g of methyl 4-hydroxybenzoate and 1 g of propyl 4-hydroxybenzoate were dissolved in 4 l of boiling purified water. In 3 l of this solution were dissolved first 10 g of 2,3-dihydroxybutanedioic acid and thereafter 20 g of the A.I. The latter solution was combined with the remaining part of the former solution and 12 l 1,2,3-propanetriol and 3 l of sorbitol 70% solution were added thereto. 40 g of sodium saccharin were dissolved in 0.5 l of water and 2 ml of raspberry and 2 ml of gooseberry essence were added. The latter solution was combined with the former, water was added q.s. to a volume of 20 l providing an oral solution

comprising 5 mg of the active ingredient per teaspoonful (5 ml). The resulting solution was filled in suitable containers.

Example 12 : CAPSULES

20 g of the A.I., 6 g sodium lauryl sulfate, 56 g starch, 56 g lactose, 0.8 g colloidal silicon dioxide, and 1.2 g magnesium stearate were vigorously stirred together. The resulting mixture was subsequently filled into 1000 suitable hardened gelatin capsules, comprising each 20 mg of the active ingredient.

Example 13 : FILM-COATED TABLETS

## Preparation of tablet core

A mixture of 100 g of the A.I., 570 g lactose and 200 g starch was mixed well and thereafter humidified with a solution of 5 g sodium dodecyl sulfate and 10 g polyvinylpyrrolidone (Kollidon-K 90 ®) in about 200 ml of water. The wet powder mixture was sieved, dried and sieved again. Then there was added 100 g microcrystalline cellulose (Avicel ®) and 15 g hydrogenated vegetable oil (Sterotex ®). The whole was mixed well and compressed into tablets, giving 10.000 tablets, each containing 10 mg of the active ingredient.

## Coating

To a solution of 10 g methyl cellulose (Methocel 60 HG®) in 75 ml of denaturated ethanol there was added a solution of 5 g of ethyl cellulose (Ethocel 22 cps ®) in 150 ml of dichloromethane. Then there were added 75 ml of dichloromethane and 2.5 ml 1,2,3-propanetriol. 10 g of polyethylene glycol was molten and dissolved in 75 ml of dichloromethane. The latter solution was added to the former and then there were added 2.5 g of magnesium octadecanoate, 5 g of polyvinylpyrrolidone and 30 ml of concentrated colour suspension (Opaspray K-1-2109®) and the whole was homogenated. The tablet cores were coated with the thus obtained mixture in a coating apparatus.

Example 14 : INJECTABLE SOLUTION

1.8 g methyl 4-hydroxybenzoate and 0.2 g propyl 4-hydroxybenzoate were dissolved in about 0.5 l of boiling water for injection. After cooling to about 50 ° C there were added while stirring 4 g lactic acid, 0.05 g propylene glycol and 4 g of the A.I.. The solution was cooled to room temperature and supplemented with water for injection q.s. ad 1 l, giving a solution comprising 4 mg/ml of A.I.. The solution was sterilized by filtration (U.S.P. XVII p. 811) and filled in sterile containers.

Example 15: SUPPOSITORIES

3 g A.I. was dissolved in a solution of 3 g 2,3-dihydroxybutanedioic acid in 25 ml polyethylene glycol 400. 12 g surfactant (SPAN®) and triglycerides (Witepsol 555 ®) q.s. ad 300 g were molten together. The latter mixture was mixed well with the former solution. The thus obtained mixture was poured into moulds at a temperature of 37-38 ° C to form 100 suppositories each containing 30 mg/ml of the A.I.

Example 16 : INJECTABLE SOLUTION

60 g of A.I. and 12 g of benzylalcohol were mixed well and sesame oil was added q.s. ad 1 l, giving a solution comprising 60 mg/ml of A.I. The solution was sterilized and filled in sterile containers.

## Claims

**1.** A compound having the formula:

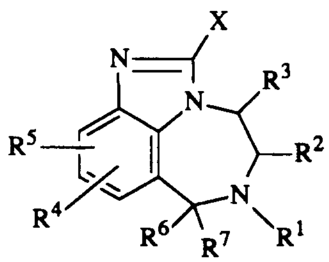

(I),

a pharmaceutically acceptable acid addition salt or a stereochemically isomeric form thereof, wherein

$R^1$ is $C_{1-6}$alkyl optionally substituted with aryl; $C_{3-6}$alkynyl; $C_{3-6}$cycloalkyl; or a radical of formula :

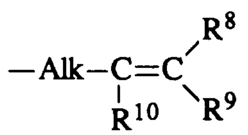

(a-1);

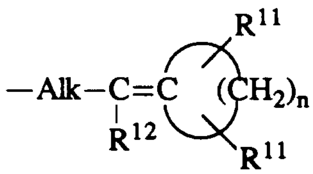

(a-2);

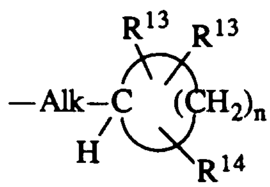

(a-3) or

$—Alk—S(O)_m—R^{15}$ (a-4);

Alk is $C_{1-6}$alkanediyl;
$R^8$ and $R^9$ each independently are hydrogen, halo, $C_{3-6}$cycloalkyl, trifluoromethyl, 2,2,2-trifluoroethyl, $C_{1-4}$alkyl optionally substituted with $C_{1-4}$alkyloxy;
$R^{10}$ is hydrogen, halo or $C_{1-4}$alkyl;
each $R^{11}$ independently is hydrogen or $C_{1-4}$alkyl; or both $R^{11}$ taken together may form a $C_{1-6}$alkanediyl radical;
$R^{12}$ is hydrogen, halo or $C_{1-4}$alkyl;
n is 2, 3, 4, 5 or 6;
each $R^{13}$ independently is hydrogen or $C_{1-4}$alkyl; or both $R^{13}$ taken together may form a $C_{1-6}$alkanediyl radical;
$R^{14}$ is hydrogen or $C_{2-6}$alkenyl;
m is 0, 1 or 2;
$R^{15}$ is $C_{1-6}$alkyl, aryl, arylmethyl, $C_{3-6}$cycloalkyl or ($C_{3-6}$cycloalkyl)$C_{1-4}$alkyl;

$R^2$ is hydrogen or $C_{1-6}$alkyl;

$R^3$ is hydrogen or $C_{1-6}$alkyl;

$R^4$ and $R^5$ each independently are hydrogen, $C_{1-6}$alkyl, halo, cyano, nitro, trifluoromethyl, hydroxy, $C_{1-6}$alkyloxy, amino, mono-or di($C_{1-6}$alkyl)amino, $C_{1-6}$alkylcarbonylamino or arylcarbonylamino;

$R^6$ is $C_{1-6}$alkyl;

$R^7$ is hydrogen;

X is OH, SH or $NR^{16}R^{17}$;
$R^{16}$ is hydrogen, $C_{1-6}$alkyl, aryl, cyano, hydroxy, amino, nitro, $C_{1-6}$alkyloxycarbonyl, $C_{1-6}$alkylcarbonyl, $C_{1-6}$alkylsulfonyl or arylsulfonyl;
$R^{17}$ is hydrogen, $C_{1-6}$alkyl or aryl; and

each aryl is phenyl optionally substituted with from 1 to 3 substituents independently selected from $C_{1-6}$alkyl, halo, hydroxy, $C_{1-6}$alkyloxy, amino, nitro and trifluoromethyl.

**2.** A compound according to claim 1 wherein $R^1$ is $C_{1-6}$alkyl, $C_{3-6}$alkenyl, $C_{3-6}$alkynyl, $C_{3-6}$cycloalkyl or $C_{1-6}$alkyl substituted with aryl or $C_{3-6}$cycloalkyl; $R^4$ and $R^5$ each independently are hydrogen, $C_{1-6}$alkyl, halo, cyano, nitro, trifluoromethyl, hydroxy, $C_{1-6}$alkyloxy, amino or mono- or di($C_{1-6}$alkyl)-amino and $R^7$ is hydrogen.

**3.** A compound according to claim 1 wherein $R^1$ is $C_{1-6}$alkyl optionally substituted with aryl; $C_{3-6}$alkynyl or a radical of formula (a-1), (a-2) or (a-3); $R^4$ and $R^5$ each independently are hydrogen, $C_{1-6}$alkyl, halo, cyano, nitro, trifluoromethyl, hydroxy or $C_{1-6}$alkyloxy.

**4.** A compound according to claim 3 wherein $R^1$ is $C_{3-6}$alkyl or a radical of formula (a-1) or (a-3); $R^5$ is hydrogen; $R^6$ is $C_{1-4}$alkyl; $R^7$ is hydrogen.

**5.** A compound according to claim 4 wherein $R^2$ and $R^3$ each independently are hydrogen or methyl; X is OH, SH; or X is $NR^{16}R^{17}$ and $R^{17}$ is hydrogen.

**6.** A compound according to claim 5 wherein X is OH or SH; $R^1$ is $C_{3-6}$alkyl or a radical of formula (a-1) wherein $R^8$ and $R^9$ each independently are $C_{3-6}$cycloalkyl, trifluoromethyl or $C_{1-4}$alkyl; or a radical of formula (a-2) wherein $R^{12}$ is hydrogen or $C_{1-4}$alkyl; or a radical of formula (a-3) wherein n is 2 or 3.

**7.** A compound according to claim 6 wherein $R^1$ is propyl; methylcyclopropyl optionally substituted with one or two methyl groups and/or a 2-methylpropenyl group; methylcyclobutyl; 2-propenyl; 2-butenyl; 2-methyl-2-butenyl; 3-methyl-2-butenyl, 2,3-dimethyl-2-butenyl or 3-ethyl-2-pentenyl; $R^4$ is hydrogen, methyl or chloro; $R^6$ is methyl.

**8.** A compound according to claim 1 wherein the compound is trans-4,5,6,7-tetrahydro-5,7-dimethyl-6-(3-methyl-2-butenyl)imidazo[4,5,1-jk][1,4]benzodiazepine-2(1H)-thione.

**9.** A pharmaceutical composition comprising a pharmaceutically acceptable carrier and as active ingredient a therapeutically effective amount of a compound as claimed in any of claims 1-8.

**10.** A process of preparing a pharmaceutical composition as claimed in claim 9, characterized in that a therapeutically effective amount of a compound as claimed in any of claims 1 to 8 is intimately mixed with a pharmaceutical carrier.

**11.** A compound as claimed in any of claims 1 to 8 for use as a medicine.

**12.** A compound having the formula :

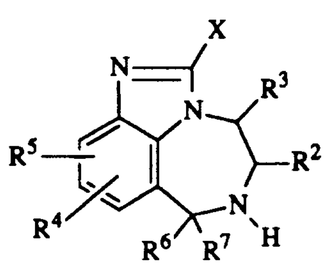

(V),

a pharmaceutically acceptable acid addition salt or a stereochemically isomeric form thereof, wherein
$R^2$ is hydrogen or $C_{1-6}$alkyl;
$R^3$ is hydrogen or $C_{1-6}$alkyl;

| | |
|---|---|
| $R^4$ and $R^5$ | each independently are hydrogen, $C_{1-6}$alkyl, halo, cyano, nitro, trifluoromethyl, hydroxy, $C_{1-6}$alkyloxy, amino, mono-or di($C_{1-6}$alkyl)amino, $C_{1-6}$alkylcarbonylamino or arylcarbonylamino; |
| $R^6$ | is $C_{1-6}$alkyl; |
| $R^7$ | is hydrogen; |
| X | is OH, SH or $NR^{16}R^{17}$; |
| $R^{16}$ | is hydrogen, $C_{1-6}$alkyl, aryl, cyano, hydroxy, amino, nitro, $C_{1-6}$alkyloxycarbonyl, $C_{1-6}$alkylcarbonyl, $C_{1-6}$alkylsulfonyl or arylsulfonyl; |
| $R^{17}$ | is hydrogen, $C_{1-6}$alkyl or aryl; and |

each aryl is phenyl optionally substituted with from 1 to 3 substituents independently selected from $C_{1-6}$alkyl, halo, hydroxy, $C_{1-6}$alkyloxy, amino, nitro and trifluoromethyl.

**13.** A compound having the formula:

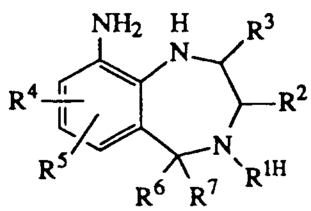

(VII-H)

an acid addition salt or a stereochemically isomeric form thereof, wherein

$R^{1H}$ is hydrogen, $C_{1-6}$alkyl optionally substituted with aryl; $C_{3-6}$alkynyl; $C_{3-6}$cycloalkyl; or a radical of formula :

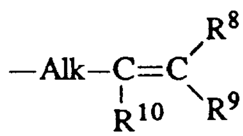

(a-1);

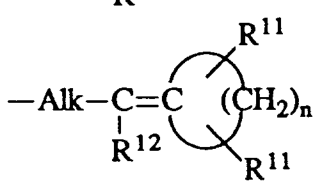

(a-2);

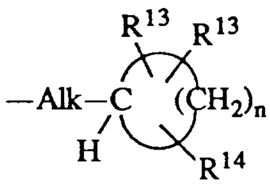

(a-3) or

—Alk—S(O)$_m$—$R^{15}$ (a-4);

Alk is $C_{1-6}$alkanediyl;
$R^8$ and $R^9$ each independently are hydrogen, halo, $C_{3-6}$cycloalkyl, trifluoromethyl, 2,2,2-trifluoroethyl, $C_{1-4}$alkyl optionally substituted with $C_{1-4}$alkyloxy;
$R^{10}$ is hydrogen, halo or $C_{1-4}$alkyl;
each $R^{11}$ independently is hydrogen or $C_{1-4}$alkyl; or both $R^{11}$ taken together may form a $C_{1-6}$alkanediyl radical;
$R^{12}$ is hydrogen, halo or $C_{1-4}$alkyl;
n is 2, 3, 4, 5 or 6;
each $R^{13}$ independently is hydrogen or $C_{1-4}$alkyl; or both $R^{13}$ taken together may form a $C_{1-6}$alkanediyl radical;
$R^{14}$ is hydrogen or $C_{2-6}$alkenyl;

m is 0, 1 or 2;
$R^{15}$ is $C_{1-6}$alkyl, aryl, arylmethyl, $C_{3-6}$cycloalkyl or ($C_{3-6}$cycloalkyl)$C_{1-4}$alkyl;

$R^2$ is hydrogen or $C_{1-6}$alkyl;

$R^3$ is hydrogen or $C_{1-6}$alkyl;

$R^4$ and $R^5$ each independently are hydrogen, $C_{1-6}$alkyl, halo, cyano, nitro, trifluoromethyl, hydroxy, $C_{1-6}$alkyloxy, amino, mono-or di($C_{1-6}$alkyl)amino, $C_{1-6}$alkylcarbonylamino or arylcarbonylamino;

$R^6$ is $C_{1-6}$alkyl;

$R^7$ is hydrogen;

each aryl is phenyl optionally substituted with from 1 to 3 substituents independently selected from $C_{1-6}$alkyl, halo, hydroxy, $C_{1-6}$alkyloxy, amino, nitro and trifluoromethyl.

**14.** A process for preparing a compound as claimed in any of claims 1 to 8, <u>characterized by</u>:

a) reacting a 4,5,6,7-tetrahydroimidazo[4,5,1-jk][1,4]benzodiazepine of formula:

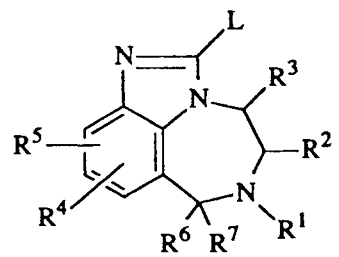

(II)

wherein $R^1$, $R^2$, $R^3$, $R^4$, $R^5$, $R^6$ and $R^7$ are as defined in formula (I) and L is a reactive leaving group with a reagent of formula B-X (III), wherein X is as defined in formula (I), in a reaction-inert solvent or in the presence of an excess of the reagent of formula (III), optionally in a reaction-inert solvent;

b) N-alkylating an intermediate of formula

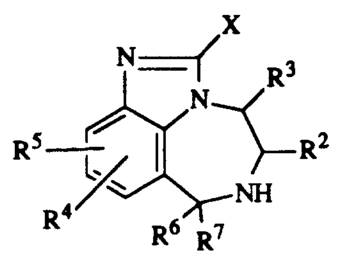

(V)

wherein $R^2$, $R^3$, $R^4$, $R^5$, $R^6$, $R^7$ and X are as defined in formula (I) with a reagent of formula $R^1$-W (IV) wherein W represents a reactive leaving group and $R^1$ is as defined in formula (I), in a reaction-inert solvent;

c) reductively N-alkylating an intermediate of formula :

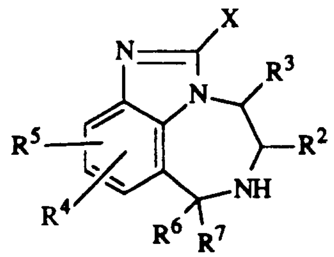

(V)

wherein $R^2$, $R^3$, $R^4$, $R^5$, $R^6$, $R^7$ and X are as defined in formula (I) with a ketone or aldehyde of formula $R^{1-b}$=O (VI), wherein $R^{1-b}$ represents a geminal bivalent radical derived from $R^{1-a}$-H, wherein two geminal hydrogen atoms are replaced by =O and $R^{1-a}$ represents $C_{1-6}$alkyl optionally

substituted with aryl; $C_{3-6}$cycloalkyl or a radical of formula (a-3); in a reaction-inert solvent, thus yielding a compound of formula :

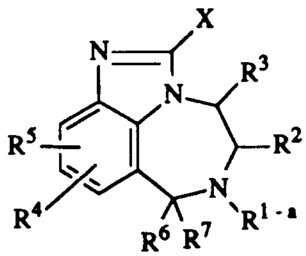

(I-a);

wherein $R^{1-a}$ is $C_{1-6}$alkyl optionally substituted with aryl; $C_{3-6}$cycloalkyl or a radical of formula (a-3) as defined in formula (I) and the carbon atom of $R^{1-a}$ adjacent to the nitrogen atom bearing $R^{1-a}$ contains at least one hydrogen atom;

d) condensing a 9-amino-2,3,4,5-tetrahydro-1H-1,4-benzodiazepine of formula :

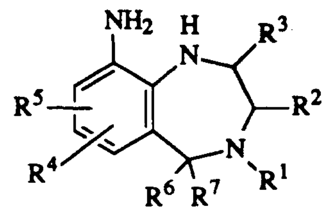

(VII)

wherein $R^1$, $R^2$, $R^3$, $R^4$, $R^5$, $R^6$ and $R^7$ are as defined in formula (I) with a reagent of formula $L^1$-C(=$X^2$)-$L^1$ (VIII) wherein $L^1$ is a reactive leaving group and $X^2$ is =O, =S or =$NR^{16}$, and $R^{16}$ is as defined in formula (I), in a reaction-inert solvent, thus yielding a compound of formula :

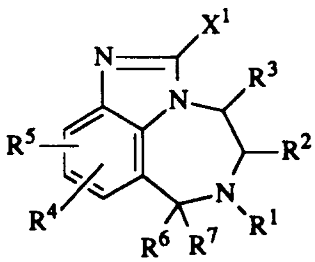

(I-b)

wherein $X^1$ is OH, SH or $NHR^{16}$;

e) thionating a compound of formula :

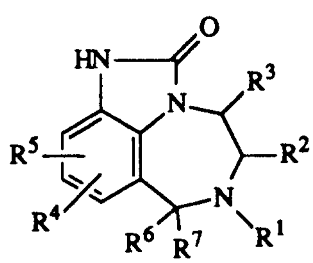

(I-b-1)

wherein $R^1$, $R^2$, $R^3$, $R^4$, $R^5$, $R^6$ and $R^7$ are as defined in formula (I) with 2,4-bis(4-methoxy-phenyl)-1,3-dithia-2,4-diphosphetane-2,4-disulfide or phosphorus pentasulfide in a reaction-inert solvent, thus yielding a compound of formula :

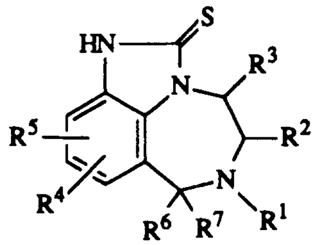

(I-b-2);

f) thiating a tetrahydroimidazo[4,5,1-jk][1,4]benzodiazepine of formula :

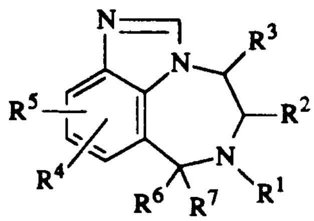

(IX)

wherein $R^1$, $R^2$, $R^3$, $R^4$, $R^5$, $R^6$ and $R^7$ are as defined in formula (I) with elemental sulfur at an elevated temperature, thus yielding a compound of formula (I-b-2);

g) reducing and thiocarbonylating a 9-nitrobenzodiazepine of formula :

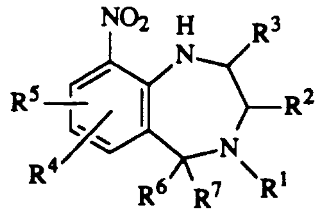

(X)

wherein $R^1$, $R^2$, $R^3$, $R^4$, $R^5$, $R^6$ and $R^7$ are as defined in formula (I) in the presence of an alkali metal sulfide or hydrogen sulfide, and carbon disulfide, thus yielding a compound of formula (I-b-2);

h) cyclizing a benzimidazole of formula

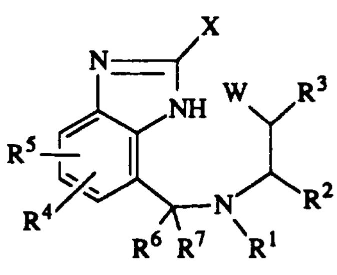

(XI)

wherein $R^1$, $R^2$, $R^3$, $R^4$, $R^5$, $R^6$, $R^7$ and X are as defined in formula (I) and W represents a reactive leaving group, in a reaction-inert solvent, and, if desired, converting the compounds of formula (I) into a therapeutically active non-toxic acid addition salt form by treatment with an acid; or conversely, converting the acid salt into the free base with alkali; and/or preparing stereochemically isomeric forms thereof.

## Patentansprüche

1. Eine Verbindung mit der Formel:

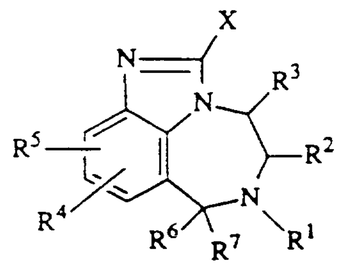

(I),

ein pharmazeutisch annehmbares Säureadditionssalz oder eine stereochemisch isomere Form hievon, worin

$R^1$ für gegebenenfalls durch Aryl substituiertes $C_{1-6}$-Alkyl; $C_{3-6}$-Alkinyl; $C_{3-6}$-Cycloalkyl; oder für einen Rest der Formel:

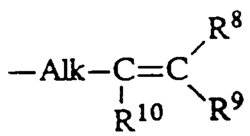

(a-1);

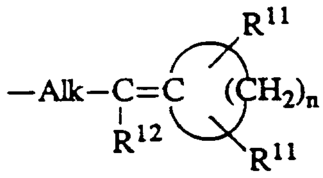

(a-2);

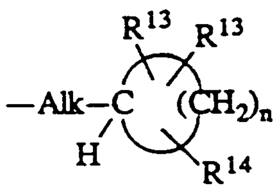

(a-3) oder

$—Alk—S(O)_m—R^{15}$ (a-4)

steht;

Alk $C_{1-6}$-Alkandiyl bezeichnet;

$R^8$ und $R^9$ jeweils unabhängig voneinander Wasserstoff, Halogen, $C_{3-6}$-Cycloalkyl, Trifluormethyl, 2,2,2-Trifluorethyl, gegebenenfalls durch $C_{1-4}$-Alkyloxy substituiertes $C_{1-4}$-Alkyl darstellen;

$R^{10}$ Wasserstoff, Halogen oder $C_{1-4}$-Alkyl bedeutet;

jeder Rest $R^{11}$ unabhängig voneinander Wasserstoff oder $C_{1-4}$-Alkyl bezeichnet; oder beide Reste $R^{11}$ zusammengenommen einen $C_{1-6}$-Alkandiylrest ausbilden können;

$R^{12}$ für Wasserstoff, Halogen oder $C_{1-4}$-Alkyl steht;

n den Wert 2, 3, 4, 5 oder 6 aufweist;

jeder Rest $R^{13}$ unabhängig voneinander Wasserstoff oder $C_{1-4}$-Alkyl bedeutet; oder beide Reste $R^{13}$ zusammengenommen einen $C_{1-6}$-Alkandiylrest ausbilden können;

$R^{14}$ Wasserstoff oder $C_{2-6}$-Alkenyl bedeutet;

m den Wert 0, 1 oder 2 aufweist;

$R^{15}$ für $C_{1-6}$-Alkyl, Aryl, Arylmethyl, $C_{3-6}$-Cycloalkyl oder ($C_{3-6}$-Cycloalkyl)$C_{1-4}$-alkyl steht;

$R^2$ Wasserstoff oder $C_{1-6}$-Alkyl bedeutet;

$R^3$ Wasserstoff oder $C_{1-6}$-Alkyl bedeutet;

$R^4$ und $R^5$ jeweils unabhängig voneinander Wasserstoff, $C_{1-6}$-Alkyl, Halogen, Cyano, Nitro, Trifluormethyl, Hydroxy, $C_{1-6}$-Alkyloxy, Amino, Mono- oder Di($C_{1-6}$-alkyl)amino, $C_{1-6}$-Alkylcarbonylamino oder Arylcarbonylamino bedeuten;

$R^6$ für $C_{1-6}$-Alkyl steht;
$R^7$ Wasserstoff bedeutet;
X für OH, SH oder $NR^{16}R^{17}$ steht;
$R^{16}$ Wasserstoff, $C_{1-6}$-Alkyl, Aryl, Cyano, Hydroxy, Amino, Nitro, $C_{1-6}$-Alkyloxycarbonyl, $C_{1-6}$-Alkylcarbonyl, $C_{1-6}$-Alkylsulfonyl oder Arylsulfonyl darstellt;
$R^{17}$ Wasserstoff, $C_{1-6}$-Alkyl oder Aryl bedeutet; und
worin jedes Aryl Phenyl bedeutet, das gegebenenfalls durch 1 bis 3 Substituenten substituiert ist, die unabhängig voneinander unter $C_{1-6}$-Alkyl, Halogen, Hydroxy, $C_{1-6}$-Alkyloxy, Amino, Nitro und Trifluormethyl ausgewählt sind.

**2.** Verbindung nach Anspruch 1, worin $R^1$ für $C_{1-6}$-Alkyl, $C_{3-6}$-Alkenyl, $C_{3-6}$-Alkinyl, $C_{3-6}$-Cycloalkyl oder durch Aryl oder $C_{3-6}$-Cycloalkyl substituiertes $C_{1-6}$-Alkyl steht; $R^4$ und $R^5$ jeweils unabhängig voneinander Wasserstoff, $C_{1-6}$-Alkyl, Halogen, Cyano, Nitro, Trifluormethyl, Hydroxy, $C_{1-6}$-Alkyloxy, Amino oder Mono- oder Di($C_{1-6}$-alkyl)amino darstellen und $R^7$ Wasserstoff bedeutet.

**3.** Verbindung nach Anspruch 1, worin $R^1$ für gegebenenfalls durch Aryl substituiertes $C_{1-6}$-Alkyl; $C_{3-6}$-Alkinyl oder einen Rest der Formel (a-1), (a-2) oder (a-3) steht; $R^4$ und $R^5$ jeweils unabhängig voneinander Wasserstoff, $C_{1-6}$-Alkyl, Halogen, Cyano, Nitro, Trifluormethyl, Hydroxy oder $C_{1-6}$-Alkyloxy bedeuten.

**4.** Verbindung nach Anspruch 3, worin $R^1$ $C_{3-6}$-Alkyl oder einen Rest der Formel (a-1) oder (a-3) darstellt; $R^5$ Wasserstoff ist; $R^6$ für $C_{1-4}$-Alkyl steht; $R^7$ Wasserstoff bedeutet.

**5.** Verbindung nach Anspruch 4, worin $R^2$ und $R^3$ jeweils unabhängig voneinander Wasserstoff oder Methyl darstellen; X für OH oder SH steht; oder X die Bedeutung $NR^{16}R^{17}$ aufweist und $R^{17}$ Wasserstoff bedeutet.

**6.** Verbindung nach Anspruch 5, worin X für OH oder SH steht; $R^1$ für $C_{3-6}$-Alkyl oder einen Rest der Formel (a-1) steht, worin $R^8$ und $R^9$ jeweils unabhängig voneinander $C_{3-6}$-Cycloalkyl, Trifluormethyl oder $C_{1-4}$-Alkyl bedeuten; oder einen Rest der Formel (a-2) bedeutet, worin $R^{12}$ Wasserstoff oder $C_{1-4}$-Alkyl darstellt; oder einen Rest der Formel (a-3) bedeutet, worin n den Wert 2 oder 3 aufweist.

**7.** Verbindung nach Anspruch 6, worin $R^1$ für Propyl; gegebenenfalls durch ein oder zwei Methylgruppen und/oder eine 2-Methylpropenylgruppe substituiertes Methylcyclopropyl; Methylcyclobutyl; 2-Propenyl; 2-Butenyl; 2-Methyl-2-butenyl; 3-Methyl-2-butenyl, 2,3-Dimethyl-2-butenyl oder 3-Ethyl-2-pentenyl steht; $R^4$ Wasserstoff, Methyl oder Chlor bedeutet; $R^6$ Methyl darstellt.

**8.** Verbindung nach Anspruch 1, worin die Verbindung trans-4,5,6,7-Tetrahydro-5,7-dimethyl-6-(3-methyl-2-butenyl)imidazo-[4,5,1-jk][1,4]benzodiazepin-2(1H)-thion ist.

**9.** Pharmazeutische Zusammensetzung, umfassend einen pharmazeutisch annehmbaren Träger und als wirksamen Bestandteil eine therapeutisch wirksame Menge einer Verbindung nach einem der Ansprüche 1 bis 8.

**10.** Verfahren zur Herstellung einer pharmazeutischen Zusammensetzung nach Anspruch 9, dadurch gekennzeichnet, daß eine therapeutisch wirksame Menge einer Verbindung nach einem der Ansprüche 1 bis 8 innig mit einem pharmazeutischen Träger vermischt wird.

**11.** Verbindung nach einem der Ansprüche 1 bis 8 zur Anwendung als ein Medikament.

**12.** Eine Verbindung mit der Formel:

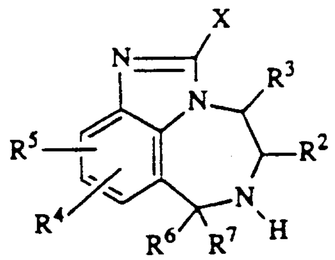

(V),

ein pharmazeutisch annehmbares Säureadditionssalz oder eine stereochemisch isomere Form hievon, worin

$R^2$ Wasserstoff oder $C_{1-6}$-Alkyl bedeutet;

$R^3$ Wasserstoff oder $C_{1-6}$-Alkyl bedeutet;

$R^4$ und $R^5$ jeweils unabhängig voneinander Wasserstoff, $C_{1-6}$-Alkyl, Halogen, Cyano, Nitro, Trifluormethyl, Hydroxy, $C_{1-6}$-Alkyloxy, Amino, Mono- oder Di($C_{1-6}$-alkyl)amino, $C_{1-6}$-Alkylcarbonylamino oder Arylcarbonylamino darstellen;

$R^6$ $C_{1-6}$-Alkyl ist;

$R^7$ Wasserstoff bedeutet;

X für OH, SH oder $NR^{16}R^{17}$ steht;

$R^{16}$ Wasserstoff, $C_{1-6}$-Alkyl, Aryl, Cyano, Hydroxy, Amino, Nitro, $C_{1-6}$-Alkyloxycarbonyl, $C_{1-6}$-Alkylcarbonyl, $C_{1-6}$-Alkylsulfonyl oder Arylsulfonyl darstellt;

$R^{17}$ Wasserstoff, $C_{1-6}$-Alkyl oder Aryl bedeutet;

und worin jedes Aryl für Phenyl steht, das gegebenenfalls durch 1 bis 3 Substituenten substituiert ist, die unabhängig voneinander unter $C_{1-6}$-Alkyl, Halogen, Hydroxy, $C_{1-6}$-Alkyloxy, Amino, Nitro und Trifluormethyl ausgewählt sind.

**13.** Eine Verbindung mit der Formel:

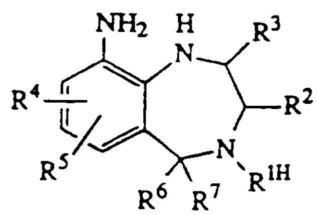

(VII-H),

ein Säureadditionssalz oder eine stereochemisch isomere Form hievon, worin

$R^{1H}$ für Wasserstoff, gegebenenfalls durch Aryl substituiertes $C_{1-6}$-Alkyl; $C_{3-6}$-Alkinyl; $C_{3-6}$-Cycloalkyl; oder für einen Rest der Formel:

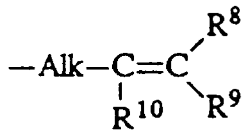

(a-1);

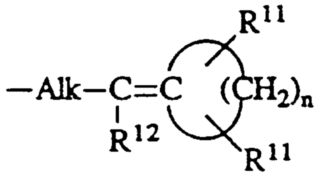

(a-2);

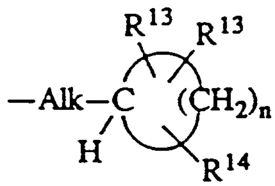

(a-3) oder

—Alk—S(O)$_m$—$R^{15}$ (a-4)

steht;

Alk für $C_{1-6}$-Alkandiyl steht;

$R^8$ und $R^9$ jeweils unabhängig voneinander Wasserstoff, Halogen, $C_{3-6}$-Cycloalkyl, Trifluormethyl, 2,2,2-Trifluorethyl, gegebenenfalls durch $C_{1-4}$-Alkyloxy substituiertes $C_{1-4}$-Alkyl darstellen;

$R^{10}$ Wasserstoff, Halogen oder $C_{1-4}$-Alkyl bedeutet;

jeder Rest $R^{11}$ unabhängig voneinander Wasserstoff oder $C_{1-4}$-Alkyl bedeutet; oder beide Reste $R^{11}$ zusammengenommen einen $C_{1-6}$-Alkandiylrest ausbilden können;

$R^{12}$ für Wasserstoff, Halogen oder $C_{1-4}$-Alkyl steht;

n den Wert 2, 3, 4, 5 oder 6 hat;

jeder Rest $R^{13}$ unabhängig voneinander für Wasserstoff oder $C_{1-4}$-Alkyl steht; oder beide Reste $R^{13}$ zusammengenommen einen $C_{1-6}$-Alkandiylrest ausbilden können;

$R^{14}$ Wasserstoff oder $C_{2-6}$-Alkenyl darstellt;

m den Wert 0, 1 oder 2 hat;

$R^{15}$ für $C_{1-6}$-Alkyl, Aryl, Arylmethyl, $C_{3-6}$-Cycloalkyl oder ($C_{3-6}$-Cycloalkyl)-$C_{1-4}$-alkyl steht;

$R^2$ Wasserstoff oder $C_{1-6}$-Alkyl bedeutet;

$R^3$ Wasserstoff oder $C_{1-6}$-Alkyl bedeutet;

$R^4$ und $R^5$ jeweils unabhängig voneinander Wasserstoff, $C_{1-6}$-Alkyl, Halogen, Cyano, Nitro, Trifluormethyl, Hydroxy, $C_{1-6}$-Alkyloxy, Amino, Mono- oder Di($C_{1-6}$-alkyl)amino, $C_{1-6}$-Alkylcarbonylamino oder Arylcarbonylamino darstellen;

$R^6$ für $C_{1-6}$-Alkyl steht;

$R^7$ Wasserstoff bedeutet;

wobei jedes Aryl Phenyl bedeutet, das gegebenenfalls durch 1 bis 3 Substituenten substituiert ist, die unabhängig voneinander unter $C_{1-6}$-Alkyl, Halogen, Hydroxy, $C_{1-6}$-Alkyloxy, Amino, Nitro und Trifluormethyl ausgewählt sind.

**14.** Verfahren zur Herstellung einer Verbindung nach einem der Ansprüche 1 bis 8, gekennzeichnet durch:

a) Umsetzen eines 4,5,6,7-Tetrahydroimidazo[4,5,1-jk]-[1,4]benzodiazepins der Formel:

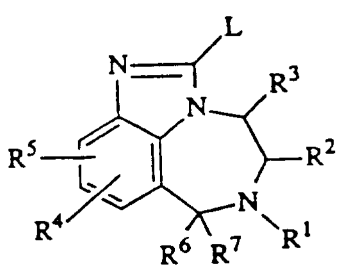

(II) ,

worin $R^1$, $R^2$, $R^3$, $R^4$, $R^5$, $R^6$ und $R^7$ wie in Formel (I) definiert sind und L eine reaktionsfähige Leaving-Gruppe darstellt, mit einem Reagens der Formel B-X (III), worin X wie in Formel (I) definiert ist, in einem reaktionsinerten Lösungsmittel oder in Anwesenheit eines Überschusses des Reagens der Formel (III), gegebenenfalls in einem reaktionsinerten Lösungsmittel;

b) N-Alkylieren eines Zwischenprodukts der Formel:

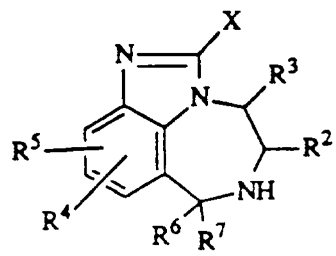

(V) ,

worin $R^2$, $R^3$, $R^4$, $R^5$, $R^6$, $R^7$ und X wie in Formel (I) definiert sind, mit einem Reagens der Formel $R^1$-W (IV), worin W eine reaktionsfähige Leaving-Gruppe darstellt und $R^1$ wie in Formel (I) definiert ist, in einem reaktionsinerten Lösungsmittel;

c) reduktives N-Alkylieren eines Zwischenprodukts der Formel:

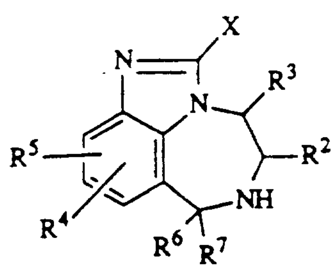

(V) ,

worin $R^2$, $R^3$, $R^4$, $R^5$, $R^6$, $R^7$ und X wie in Formel (I) definiert sind, mit einem Keton oder Aldehyd der Formel $R^{1-b}=O$ (VI), worin $R^{1-b}$ einen geminalen zweiwertigen Rest bedeutet, der von $R^{1-a}$-H abgeleitet ist, worin zwei geminale Wasserstoffatome durch =O ersetzt sind und $R^{1-a}$ für gegebenenfalls durch Aryl substituiertes $C_{1-6}$-Alkyl; $C_{3-6}$-Cycloalkyl oder für einen Rst der Formel (a-3) steht; in einem reaktionsinerten Lösungsmittel unter Ausbildung einer Verbindung der Formel:

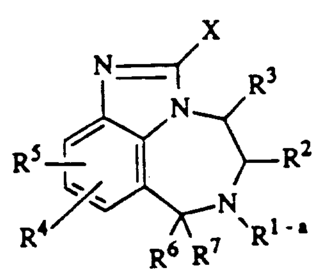

(I-a);

worin $R^{1-a}$ für gegebenenfalls durch Aryl substituiertes $C_{1-6}$-Alkyl; $C_{3-6}$-Cycloalkyl oder für einen Rest der Formel (a-3), wie in Formel (I) definiert, steht und das Kohlenstoffatom von $R^{1-a}$, das dem den Rest $R^{1-a}$ tragenden Stickstoffatom benachbart ist, wenigstens ein Wasserstoffatom enthält;

d) Kondensieren eines 9-Amino-2,3,4,5-tetrahydro-1H-1,4-benzodiazepins der Formel:

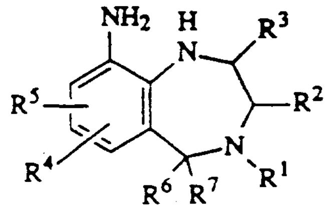

(VII) ,

worin $R^1$, $R^2$, $R^3$, $R^4$, $R^5$, $R^6$ und $R^7$ wie in Formel (I) definiert sind, mit einem Reagens der Formel $L^1$-C(=$X^2$)-$L^1$ (VIII), worin $L^1$ eine reaktionsfähige Leavinggruppe darstellt und $X^2$ für =O, =S oder =$NR^{16}$ steht und $R^{16}$ wie in Formel (I) definiert ist, in einem reaktionsinerten Lösungsmittel unter Ausbildung einer Verbindung der Formel:

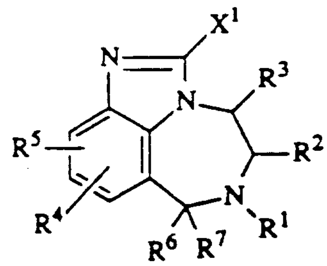

(I-b) ,

worin $X^1$ für OH, SH oder $NHR^{16}$ steht;

e) Thionieren einer Verbindung der Formel:

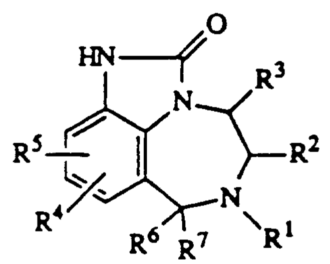

(I-b-1) ,

worin $R^1$, $R^2$, $R^3$, $R^4$, $R^5$, $R^6$ und $R^7$ wie in Formel (I) definiert sind, mit 2,4-Bis(4-methoxyphenyl)-1,3-dithia-2,4-diphosphetan-2,4-disulfid oder Phosphorpentasulfid in einem reaktionsinerten Lösungsmittel unter Ausbildung einer Verbindung der Formel:

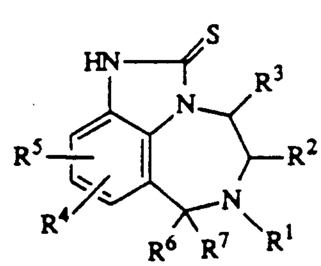

(I-b-2) ;

f) Thiatieren eines Tetrahydroimidazo[4,5,1-jk][1,4]-benzodiazepins der Formel:

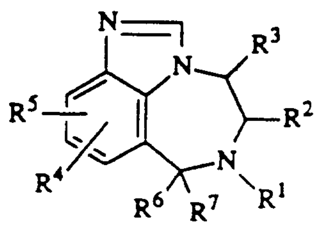

(IX) ,

worin $R^1$, $R^2$, $R^3$, $R^4$, $R^5$, $R^6$ und $R^7$ wie in Formel (I) definiert sind, mit elementarem Schwefel bei erhöhter Temperatur unter Ausbildung einer Verbindung der Formel (I-b-2);

g) Reduzieren und Thiocarbonylieren eines 9-Nitrobenzodiazepins der Formel:

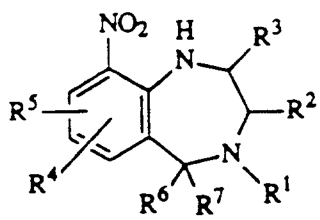

(X) ,

worin $R^1$, $R^2$, $R^3$, $R^4$, $R^5$, $R^6$ und $R^7$ wie in Formel (I) definiert sind, in Anwesenheit eines Alkalimetallsulfids oder -hydrogensulfids und von Schwefelkohlenstoff unter Ausbildung einer Verbindung der Formel (I-b-2);

h) Cyclisieren eines Benzimidazols der Formel

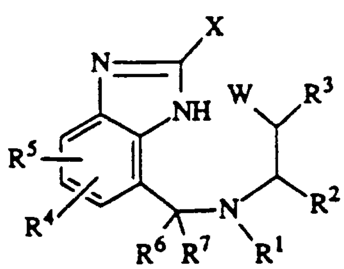

(XI) ,

worin $R^1$, $R^2$, $R^3$, $R^4$, $R^5$, $R^6$, $R^7$ und X wie in Formel (I) definiert sind und W eine reaktionsfähige Leaving-Gruppe darstellt, in einem reaktionsinerten Lösungsmittel, und gewünschtenfalls Überführen der Verbindungen der Formel (I) in eine therapeutisch wirksame nicht-toxische Säureadditionssalzform durch Behandlung mit einer Säure; oder umgekehrt Überführen des Säuresalzes in die freie Base mit Alkali; und/oder Bereiten stereochemisch isomerer Formen hievon.

## Revendications

**1.** Composé de formule :

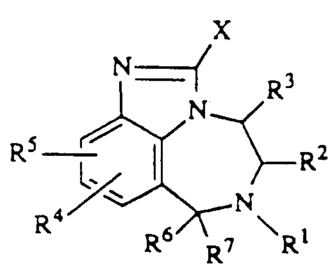

(I),

un de ces sels d'addition d'acide pharmaceutiquement acceptable ou forme stéréochimiquement isomérique, où

$R^1$ est un alkyle en $C_1$ à $C_6$ facultativement substitué par un aryle; un alcynyle en $C_3$ à $C_6$; un cycloalkyle en $C_3$ à $C_6$; ou un radical de formule :

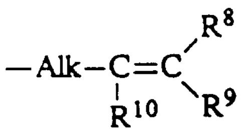
(a-1);

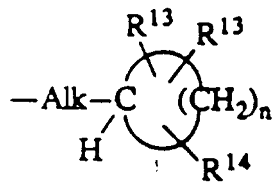
(a-3) ou

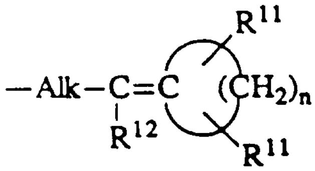
(a-2);

$-Alk-S(O)_m-R^{15}$ (a-4);

Alk représente un alcanediyle en $C_1$ à $C_6$;
$R^8$ et $R^9$ représente chacun indépendamment un hydrogène, halo, cycloakyle en $C_3$ à $C_6$, trifluorométhyle, 2,2,2-trifluoroéthyle, alkyle en $C_1$ à $C_4$, facultativement substitué par un alkyloxy en $C_1$ à $C_4$;
$R^{10}$ représente un hydrogène, halo ou alkyle en $C_1$ à $C_4$;
chaque $R^{11}$ représente indépendamment un hydrogène ou un alkyle en $C_1$ à $C_4$; où les deux $R^{11}$ pris ensemble peuvent former un radical alcanediyle en $C_1$ à $C_6$;
$R^{12}$ est un hydrogène, halo ou alkyle en $C_1$ à $C_4$;
n vaut 2, 3, 4, 5 ou 6;
chaque $R^{13}$ représente indépendamment un hydrogène ou un alkyle en $C_1$ à $C_4$; où les deux $R^{13}$ pris ensemble peuvent former un radical alcanediyle en $C_1$ à $C_6$;
$R^{14}$ est un hydrogène ou un alcényle en $C_2$ à $C_6$;
m vaut 0, 1 ou 2;
$R^{15}$ est un alkyle en $C_1$ à $C_6$, aryle, arylméthyle, cycloalkyle en $C_3$ à $C_6$ ou (cycloalkyle en $C_3$ à $C_6$)alkyle en $C_1$ à $C_4$;

$R^2$ est un hydrogène ou un alkyle en $C_1$ à $C_6$;
$R^3$ est un hydrogène ou un alkyle en $C_1$ à $C_6$;
$R^4$ et $R^5$ représentent chacun indépendamment un hydrogène, alkyle en $C_1$ à $C_6$, halo, cyano, nitro, trifluorométhyle, hydroxy, alkyloxy en $C_1$ à $C_6$, amino, mono- ou di(alkyle en $C_1$ à $C_6$) amino, alkyle en $C_1$ à $C_6$-carbonylamino ou arylcarbonylamino;
$R^6$ est un alkyle en $C_1$ à $C_6$;
$R^7$ est un hydrogène;
X représente OH, SH ou $NR^{16}R^{17}$;
$R^{16}$ est un hydrogène, alkyle en $C_1$ à $C_6$, aryle, cyano, hydroxy, amino, nitro, alkyloxy en $C_1$ à $C_6$-carbonyle, alkyle en $C_1$ à $C_6$-carbonyle, alkyle en $C_1$ à $C_6$-sulfonyle ou arylsulfonyle;
$R^{17}$ est un hydrogène, alkyle en $C_1$ à $C_6$ ou aryle; et

chaque aryle est un phényle facultativement substitué par de 1 à 3 substituants choisis indépendamment parmi alkyle en $C_1$ à $C_6$, halo, hydroxy, alkyloxy en $C_1$ à $C_6$, amino, nitro et trifluorométhyle.

**2.** Composé selon la revendication 1, dans lequel $R^1$ est un alkyle en $C_1$ à $C_6$, alcényle en $C_3$ à $C_6$, alcynyle en $C_3$ à $C_6$, cycloakyle en $C_3$ à $C_6$ ou alkyle en $C_1$ à $C_6$ substitué par un aryle ou un cycloalkyle en $C_3$ à $C_6$; $R^4$ et $R^5$ représentent chacun indépendamment un hydrogène, alkyle en $C_1$ à $C_6$, halo, cyano, nitro, trifluorométhyle, hydroxy, alkyloxy en $C_1$ à $C_6$, amino ou mono- ou di(alkyle en $C_1$ à $C_6$)amino; $R^7$ est un hydrogène; et $R^2$, $R^3$, X, $R^{16}$, $R^{17}$ sont tels que définis sous la formule (I).

**3.** Composé selon la revendication 1, dans lequel $R^1$ est un alkyle en $C_1$ à $C_6$ facultativement substitué par un aryle, un alcynyle en $C_3$ à $C_6$ ou un radical de formule (a-1), (a-2) ou (a-3); $R^4$ et $R^5$ représentent chacun indépendamment un hydrogène, un alkyle en $C_1$ à $C_6$, un halo, un cyano, un nitro,

un trifluorométhyle, un hydroxy ou un alkyloxy en $C_1$ à $C_6$.

**4.** Composé selon la revendication 3, où $R^1$ est un alkyle en $C_3$ à $C_6$ ou un radical de formule (a-1) ou (a-3); $R^5$ est un hydrogène; $R^6$ est un alkyle en $C_1$ à $C_4$; $R^7$ est un hydrogène.

**5.** Composé selon la revendication 4, où $R^2$ et $R^3$ représentent chacun indépendamment un hydrogène ou un méthyle; X représente OH ou SH, où X représente $NR^{16}R^{17}$ et $R^{17}$ est un hydrogène.

**6.** Composé selon la revendication 5, où X représente OH ou SH; $R^1$ est un alkyle en $C_3$ à $C_6$ ou un radical formule (a-1) où $R^8$ et $R^9$ représentent chacun indépendamment un cycloalkyle en $C_3$ à $C_6$, un trifluorométhyle ou un alkyle en $C_1$ à $C_4$; ou un radical de formule (a-2) où $R^{12}$ est un hydrogène ou un alkyle en $C_1$ à $C_4$; ou un radical de formule (a-3) où n vaut 2 ou 3.

**7.** Composé selon la revendication 6, où $R^1$ est un propyle; un méthylcyclopropyle facultativement substitué par un ou deux groupes méthyle et/ou un groupe 2-méthylpropényle; un méthylcyclobutyle; un 2-propényle; un 2-buténysle; un 2-méthyl-2-buténysle; un 3-méthyl-2-buténysle; un 2,3-diméthyl-2-buténysl ou un 3-éthyl-2-penténysle; et/ou $R^4$ est un hydrogène, méthyle ou chloro; et/ou $R^6$ est un méthyle.

**8.** Composé selon la revendication 1, dans lequel le composé est la trans-4,5,6,7-tétrahydro-5,7-diméthyl-6-(3-méthyl-2-butényl) imidazo [4,5,1-jk][1,4]benzodiazépine-2(1H)-thione.

**9.** Composition pharmaceutique comprenant un support pharmaceutiquement acceptable et comme ingrédient actif une quantité thérapeutiquement efficace d'un composé selon l'une quelconque des revendications 1-8.

**10.** Procédé de préparation d'une composition pharmaceutique selon la revendication 9, caractérisé en ce qu'on mélange intimement avec un support pharmaceutique une quantité thérapeutiquement efficace d'un composé selon l'une quelconque des revendications 1 à 8.

**11.** Composé selon l'une quelconque des revendications 1 à 8 pour utilisation comme médicament.

**12.** Composé de formule :

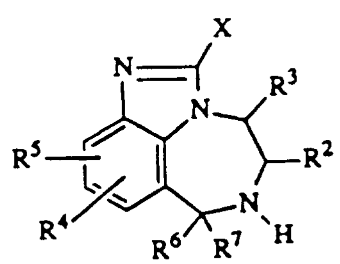

(V),

un de ses sels d'addition d'acide pharmaceutiquement acceptable ou forme stéréochimiquement isomérique, dans laquelle

| | |
|---|---|
| $R^2$ | est un hydrogène ou un alkyle en $C_1$ à $C_6$; |
| $R^3$ | est un hydrogène ou un alkyle en $C_1$ à $C_6$; |
| $R^4$ et $R^5$ | représentent chacun indépendamment un hydrogène, alkyle en $C_1$ à $C_6$, halo, cyano, nitro, trifluorométhyle, hydroxy, un alkyloxy en $C_1$ à $C_6$, un amino, un mono- ou di-(alkyle en $C_1$ à $C_6$) amino, un alkyle en $C_1$ à $C_6$-carbonylamino ou arylcarbonylamino; |
| $R^6$ | est un alkyle en $C_1$ à $C_6$; |
| $R^7$ | est un hydrogène; |
| X | représente OH, SH ou $NR^{16}R^{17}$; |
| $R^{16}$ | est un hydrogène, un alkyle en $C_1$ à $C_6$, un aryle, cyano, hydroxy, amino, nitro, alkyloxy en $C_1$ à $C_6$-carbonyle, alkyle en $C_1$ à $C_6$-carbonyle, alkyle en $C_1$ à $C_6$-sulfonyle ou arylsulfonyle; |
| $R^{17}$ | est un hydrogène, un alkyle en $C_1$ à $C_6$ ou un aryle; et |

chaque aryle est un phényle facultativement substitué par de 1 à 3 substituants choisis indépendam-

ment parmi alkyle en $C_1$ à $C_6$, halo, hydroxy, alkyle en $C_1$ à $C_6$, amino, nitro et trifluorométhyle.

**13.** Composé de formule

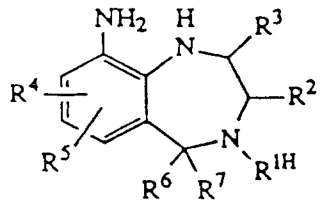

(VII-H)

un de ces sels d'addition d'acide ou forme stéréochimiquement isomé_rique, dans laquelle $R^{1H}$ est un hydrogène, un alkyle en $C_1$ à $C_6$ facultativement substitué par un aryle; un alcynyle en $C_3$ à $C_6$; un cycloalkyle en $C_3$ à $C_6$ ou un radical de formule

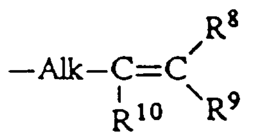

(a-1);

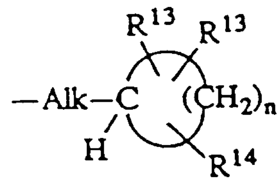

(a-3) ou

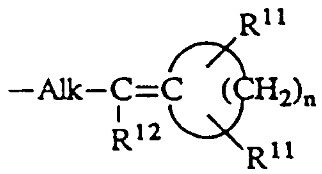

(a-2);

$-Alk-S(O)_m-R^{15}$ (a-4);

Alk est un alcanediyle en $C_{1-6}$;
$R^8$ et $R^9$ représentent chacun indépendamment un hydrogène, halo, cycloakyle en $C_3$ à $C_6$, trifluorométhyle, 2,2,2-trifluoroéthyle, alkyle en $C_1$ à $C_4$ facultativement substitué par un alkyloxy en $C_1$ à $C_4$;
$R^{10}$ est un hydrogène, halo ou alkyle en $C_1$ à $C_4$;
chaque $R^{11}$ représente indépendamment un hydrogène ou un alkyle en $C_1$ à $C_4$;
ou les deux $R^{11}$ pris ensemble peuvent former un radical alcanediyle en $C_1$ à $C_6$;
$R^{12}$ est un hydrogène, halo ou alkyle en $C_1$ à $C_4$;
n vaut 2, 3, 4, 5 ou 6;
chaque $R^{13}$ représente indépendamment un hydrogène ou un alkyle en $C_1$ à $C_4$; ou les deux $R^{13}$ pris ensemble peuvent former un radical alcanediyle en $C_1$ à $C_6$;
$R^{14}$ est un hydrogène ou un alcényle en $C_2$ à $C_6$;
m vaut 0, 1 ou 2;
$R^{15}$ est un alkyle en $C_1$ à $C_6$, aryle, arylméthyle, cycloalkyle en $C_3$ à $C_6$ ou (cycloalkyle en $C_3$ à $C_6$)-alkyle en $C_1$ à $C_4$;

| | |
|---|---|
| $R^2$ | est un hydrogène ou un alkyle en $C_1$ à $C_6$; |
| $R^3$ | est un hydrogène ou un alkyle en $C_1$ à $C_6$; |
| $R^4$ et $R^5$ | représentent chacun indépendamment un hydrogène, alkyle en $C_1$ à $C_6$, halo, cyano, nitro, trifluorométhyle, hydroxy, alkyloxy en $C_1$ à $C_6$, amino, mono- ou di(alkyle en $C_1$ à $C_6$) amino, alkyle en $C_1$ à $C_6$-carbonylamino ou arylcarbonylamino; |
| $R^6$ | est un alkyle en $C_1$ à $C_6$; |
| $R^7$ | est un hydrogène; chaque aryle est un phényle facultativement substitué par de 1 à 3 substituants choisis indépendamment parmi alkyle en $C_1$ à $C_6$, halo, hydroxy, alkyloxy en $C_1$ à $C_6$, amino, nitro et trifluorométhyle. |

**14.** Procédé de préparation d'un composé selon l'une quelconque des revendications 1 à 8, caractérisé en ce que :

a) on fait réagir une 4,5,6,7-tétrahydroimidazo[4,5,1-jk][1,4]benzodiazépine de formule

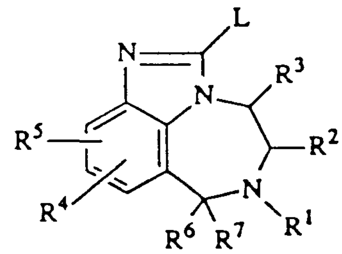

(II)

dans laquelle $R^1$, $R^2$, $R^3$, $R^4$, $R^5$, $R^6$ et $R^7$ sont tels que définis dans la formule (I) et L est un groupe sortant et actif avec un réactif de formule B-X (III), où X est tel que défini dans la formule (I), dans un solvant inerte vis-à-vis de la réaction ou en présence d'un excès du réactif de formule (III), facultativement dans un solvant inerte vis-à-vis de la réaction;

b) On N-alkyle l'intermédiaire de formule

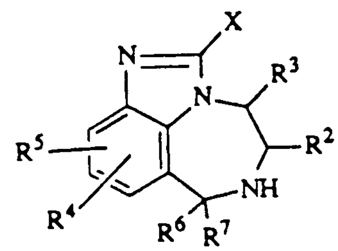

(V)

dans laquelle $R^2$, $R^3$, $5^4$, $R^5$, $R^6$, $R^7$ et X sont tels que définis dans la formule (I) avec un réactif de formule $R^1$-W(IV) où W représente un groupe sortant et actif et $R^1$ est tel que défini dans la formule (I), dans un solvant inerte vis-à-vis de la réaction;

c) on procède à une N-alkylation réductrice d'un intermédiaire de formule :

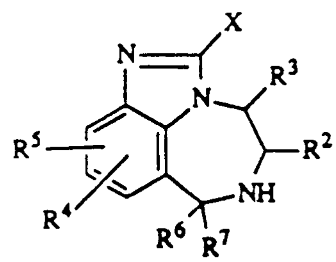

(V)

dans laquelle $R^2$, $R^3$,$R^4$, $R^5$, $R^6$, $R^7$ et X sont tels que définis dans la formule (I) avec une cétone ou un aldéhyde de formule $R^{1-b}=O$ (VI), ou $R^{1-b}$ représente un radical bivalent géminé dérivé de $R^{1-a}$-H, où deux atomes d'hydrogène géminé sont remplacés par $=O$ et $R^{1-a}$ représente un alkyle en $C_1$ à $C_6$ facultativement substitué par un aryle; un cycloalkyle en $C_3$ à $C_6$ ou un radical de formule (a-3); dans un solvant inerte vis-à-vis de la réaction, donnant ainsi un composé de formule :

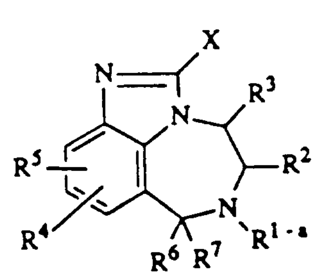

(I-a);

dans lequel $R^{1-a}$ est un alkyle en $C_1$ à $C_6$ facultativement substitué par un aryle; un cycloalkyle en

$C_3$ à $C_6$ ou un radical de formule (a-3) tel que défini dans la formule (I) et l'atome de carbone de $R^{1-a}$ adjacent à l'atome d'azote portant $R^{1-a}$ contient au moins un atome d'hydrogène;

d) on condense une 9-amino-2,3,4,5-tétrahydro-1H-1,4-benzodiazépine de formule :

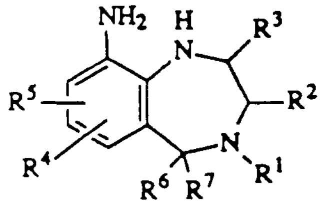

(VII)

dans laquelle $R^2$, $R^3$,$R^4$, $R^5$, $R^6$ et $R^7$ ont été définis dans la formule (I) avec un réactif de formule $L^1$-C(=$X^2$)-$L^1$ (VIII) où $L^1$ est un groupe sortant et actif et $X^2$ représente =O, =S ou $NR^{16}$, et $R^{16}$ est tel que défini dans la formule (I), dans un solvant inerte vis-à-vis de la réaction, donnant ainsi un composé de formule

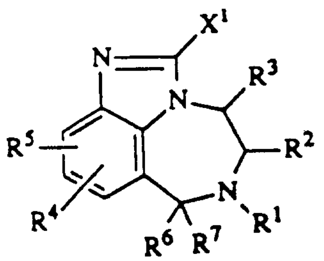

(I-b)

dans laquelle $X^1$ représente OH, SH ou $NHR^{16}$;

e) on thione un composé de formule :

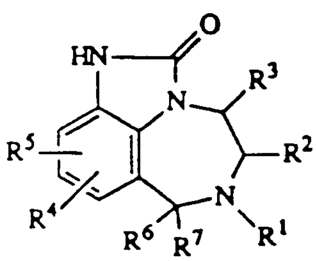

(I-b-1)

dans laquelle $R^1$, $R^2$, $R^3$, $R^4$, $R^5$, $R^6$ et $R^7$ sont tels que définis dans la formule (I) avec le 2,4-bis(4-méthoxy-phényl)-1,3-dithia-2,4-diphosphétane-2,4-disulfure ou le pentasulfure de phosphore dans un solvant inerte vis-à-vis de la réaction, donnant ainsi un composé de formule :

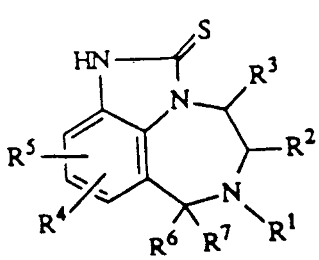

(I-b-2) ;

f) on thiate une tétrahydroimidazo[4,5,1-jk][1,4] benzodiazépine de formule

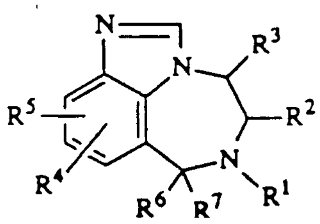

(IX)

dans laquelle $R^1$, $R^2$, $R^3$, $R^4$, $R^5$, $R^6$ et $R^7$ sont tels que définis dans la formule (I), avec du soufre élémentaire à une température élevée, donnant ainsi un composé de formule (I-b-2);

g) on réduit et on thiocarbonyle une 9-nitrobenzodiazépine de formule :

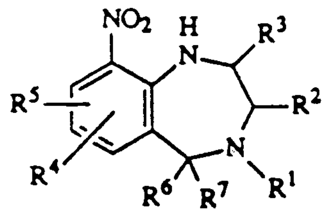

(X)

dans laquelle $R^1$, $R^2$, $R^3$,$R^4$, $R^5$, $R^6$ et $R^7$ sont tels que définis dans la formule (I) en présence d'un sulfure de métal alcalin ou d'acide sulfurique, et de disulfure de carbone, donnant ainsi un composé de formule (I-b-2);

h) on cyclise un benzimidazole de formule

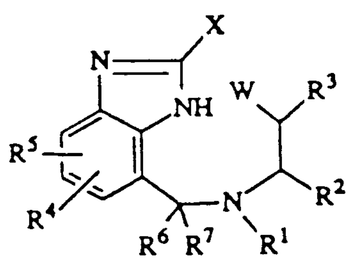

(XI)

dans laquelle $R^1$, $R^2$, $R^3$,$R^4$, $R^5$, $R^6$, $R^7$ et X sont tels que définis dans la formule (I) et W représente un groupe sortant réactif, dans un solvant inerte vis-à-vis de la réaction et, si on le désire, on transforme les composés de formule (I) en une forme sel d'addition d'acide non toxique thérapeutiquement active par traitement avec un acide; ou inversement, on transforme le sel d'acide en la base libre avec une base; et/ou on prépare leur forme stéréochimiquement isomérique.